# EUROPEAN PATENT SPECIFICATION

(11) **EP 4 291 144 B1**
(45) Date of publication and mention of the grant of the patent: **15.07.2026**
(21) Application number: 22709368.9
(22) Date of filing: 08.02.2022
(51) Int. Cl.: A61F 9/00

(54) **SUPRACHOROIDAL INJECTION DEVICE**
SUPRACHOROIDALE INJEKTIONSVORRICHTUNG
DISPOSITIF D'INJECTION SUPRACHOROÏDIEN

(30) Priority: 09.02.2021 US 202163147410 P; 11.10.2021 US 202163254262 P
(43) Date of publication of application: 20.12.2023
(73) Proprietor: Genentech, Inc., South San Francisco, CA 94080-4990 (US)
(72) Inventor: HEDGELAND, Mark, King Of Prussia, PA 19406 (US); MEYER, Thomas, E., King of Prussia, PA 19406 (US); CREEL, Justin, A., King of Prussia, PA 19406 (US)
(74) Representative: Carpmaels & Ransford LLP
(86) International application number: PCT/IB2022/000064
(87) International publication number: WO 2022/172086

(56) References cited:
- WO-A1-2008/084063
- WO-A1-2016/083669
- WO-A1-2016/196841
- WO-A2-2012/051575
- US-A1- 2013 216 623
- US-B1- 7 678 078

## Description

### BACKGROUND

The human eye comprises several layers. The white outer layer is the sclera, which surrounds the choroid layer. The region between the sclera and the choroid layer may be referred to as the suprachoroidal space, though the sclera and choroid layer may be in direct apposition with each other. The retina is interior to the choroid layer. The sclera contains collagen and elastic fiber, providing protection to the choroid and retina. The choroid layer includes vasculature providing oxygen and nourishment to the retina. The retina comprises light sensitive tissue, including rods and cones. The region between the choroid and the retina may be referred to as the subretinal space, though the choroid and the retina may be in direct apposition with each other. The vitreous humor is a gel-like tissue contained in the largest interior region of the eye (i.e., the vitreous chamber), interior to the retina.

In some scenarios, it may be desirable to dispense a therapeutic agent to a patient's eye to treat one or more ocular conditions. Such ocular conditions may include, by way of example only, macular degeneration, retinitis pigmentosa, diabetic retinopathy, and/or other ocular conditions. The dispensed therapeutic agent may comprise various kinds of drugs including but not limited to small molecules, large molecules, cells, and/or gene therapies, etc. As described in U.S. Pat. No. 10,226,379, entitled "Method and Apparatus for Subretinal Administration of Therapeutic Agent," issued March 12, 2019, a therapeutic agent may be administered to the subretinal space (i.e., the interstitial region between the choroid and the retina). Alternatively, a therapeutic agent may be administered to the suprachoroidal space (i.e., the interstitial region between the sclera and the choroid) or to the vitreous region of the eye.

WO2016/196841A1 discloses a device for placing an intraocular shunt *ab externo* into an eye. WO2008/084063A1 discloses an apparatus for intraocular injection. US7678078B1 discloses an intravitreal injection device for administering a pharmacological agent formulation to an intravitreal compartment of an eye. WO2016/083669A1 discloses an ocular therapeutics tool comprising a stabilizer comprising a hollow body and a base connected to the body, the hollow body extending from the base, and the base being adapted to fit the eye surface, and an injection guide connectable to the stabilizer and adapted to receive an injection needle, wherein the injection guide comprises at least one stopper adapted to define the injection depth of a needle inserted into the injection guide. US2013/216623A1 discloses apparatus and formulations for suprachoroidal drug delivery. WO2012/051575A2 discloses devices for accessing the suprachoroidal space or sub-retinal space in an eye.

While a variety of surgical methods and instruments have been made and used to treat an eye, it is believed that no one prior to the inventors has made or used the invention described in the appended claims.

### SUMMARY OF THE INVENTION

The present invention provides an apparatus for delivering fluid from a syringe to a suprachoroidal space as recited in claim 1. Optional features are recited in the dependent claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an example of an instrument for delivery of a therapeutic agent to a suprachoroidal space;
FIG. 2 depicts a perspective view of a head of the instrument of FIG. 1;
FIG. 3 depicts another perspective view of the head of FIG. 2;
FIG. 4 depicts a front elevation view of the head of FIG. 2;
FIG. 5 depicts a side cross-sectional view of the head of FIG. 2, taken along 5-5 of FIG. 4;
FIG. 6 depicts a side elevation view of the instrument of FIG. 1, with the head of FIG. 2 engaged with a schematic representation of an eye of a patient;
FIG. 7 depicts a side cross-sectional view of the head of FIG. 2 engaged with a schematic representation of an eye of a patient;
FIG. 8A depicts a side cross-sectional view of another example of a head that may be incorporated into the instrument of FIG. 1, with the head engaging a schematic representation of an eye of a patient, and with a needle in a retracted position;
FIG. 8B depicts a side-cross-sectional view of the head of FIG. 8A engaging a schematic representation of an eye of a patient, and with the needle in an advanced position;
FIG. 9A depicts a side cross-sectional view of another example of a head that may be incorporated into the instrument of FIG. 1, with the head engaging a schematic representation of an eye of a patient, and with a needle in a retracted position;
FIG. 9B depicts a side-cross-sectional view of the head of FIG. 9A engaging a schematic representation of an eye of a patient, and with the needle in an advanced position;
FIG. 10 depicts a side elevation view of another example of a head that may be incorporated into the instrument of FIG. 1, with a needle protruding distally from the head;
FIG. 11 depicts a side elevation view of the needle of FIG. 10 disposed in a schematic representation of an eye of a patient, and with the needle in several alternative use positions;
FIG. 12 depicts a schematic view of another example of a head that may be incorporated into the instrument of FIG. 1;
FIG. 13 depicts a plot of voltage as a function of needle depth associated with an example of use of the head of FIG. 12;
FIG. 14 depicts a side elevation view of another example of a head that may be incorporated into the instrument of FIG. 1, with a needle of the head disposed in layers of an eye of a patient;
FIG. 15 depicts a side elevation view of another example of a head that may be incorporated into the instrument of FIG. 1, with a needle of the head disposed in layers of an eye of a patient;
FIG. 16 depicts a perspective view of an embodiment of an instrument for delivery of a therapeutic agent to a suprachoroidal space according to the invention;
FIG. 17 depicts a side cross-sectional view of a head of the instrument of FIG. 16 engaged with a schematic representation of an eye of a patient;
FIG. 18A depicts a schematic view of a first stage of a procedure where the instrument of FIG. 16 is used to deliver therapeutic agent to a suprachoroidal space;
FIG. 18B depicts a schematic view of a second stage of a procedure where the instrument of FIG. 16 is used to deliver therapeutic agent to a suprachoroidal space;
FIG. 18C depicts a schematic view of a third stage of a procedure where the instrument of FIG. 16 is used to deliver therapeutic agent to a suprachoroidal space;
FIG. 19 depicts a side cross-sectional view of the head of the instrument of FIG. 16 engaged with a schematic representation of an eye of a patient, with a needle disposed in a thin sclera layer; and
FIG. 20 depicts a side cross-sectional view of the head of the instrument of FIG. 16 engaged with a schematic representation of an eye of a patient, with a needle disposed in a thick sclera layer.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

For clarity of disclosure, the terms "proximal" and "distal" are defined herein relative to a surgeon or other operator grasping a surgical instrument having a distal surgical end effector. The term "proximal" refers the position of an element closer to the surgeon or other operator and the term "distal" refers to the position of an element closer to the surgical end effector of the surgical instrument and further away from the surgeon or other operator.

### I. Suprachoroidal Administration of Therapeutic Agent

As noted above, there may be scenarios where it is desirable to administer a therapeutic agent to an eye of a patient. By way of example only, this may be done to treat one or more ocular conditions such as macular degeneration, retinitis pigmentosa, diabetic retinopathy, other retinal disease, and/or other ocular conditions. The dispensed therapeutic agent may comprise various kinds of drugs including but not limited to small molecules, large molecules, cells, and/or gene therapies, etc.

Another variable in dispensing a therapeutic agent to an eye of a patient is the precise location in the eye in which to deliver the therapeutic agent. In determining the appropriate location, it may be necessary to identify where to insert a needle into the eye (e.g., somewhere between the limbus and the equator of the eye), as the needle insertion site may affect the efficacy of delivery and also the risk of trauma posed by the needle to various structures within the eye. Another potentially critical factor in determining the appropriate location for delivering a therapeutic agent to the eye may include the depth of insertion of the needle into the eye. For instance, it may be desirable to insert the needle such that the needle will deliver the therapeutic agent to the suprachoroidal space, to the subretinal space, to the vitreous region, or elsewhere in the eye. This delivery location and corresponding needle insertion depth may also affect the efficacy of delivery and the risk of trauma posed by the needle to various structures within the eye.

Given the relatively small size of anatomical structures within the eye, the thinness of the layers in the eye, and the procedural sensitivity to the instrument reaching the desired delivery location, it may be desirable to provide a delivery instrument that is configured to consistently promote reliable delivery of therapeutic agents to the appropriate location in the eye. In other words, it may be desirable to provide a delivery instrument that is less sensitive to the expertise and technique of an operator who might otherwise attempt to deliver the therapeutic agent using a conventional delivery instrument such as a conventional syringe. The following description provides several examples of delivery instruments that may be used to deliver a therapeutic agent to a precise target location in a patient's eye with consistency and reliability, reducing the amount of operator skill that might otherwise be necessary to reliably reach the target location with a conventional delivery instrument. While the examples described below provide delivery at a particular region between the limbus and equator of the eye, and to the suprachoroidal space of the eye, the instrument may be modified to provide delivery at any other suitable location in the eye.

### A. Example of Instrument with Fixed Position Needle and Integral Plunger

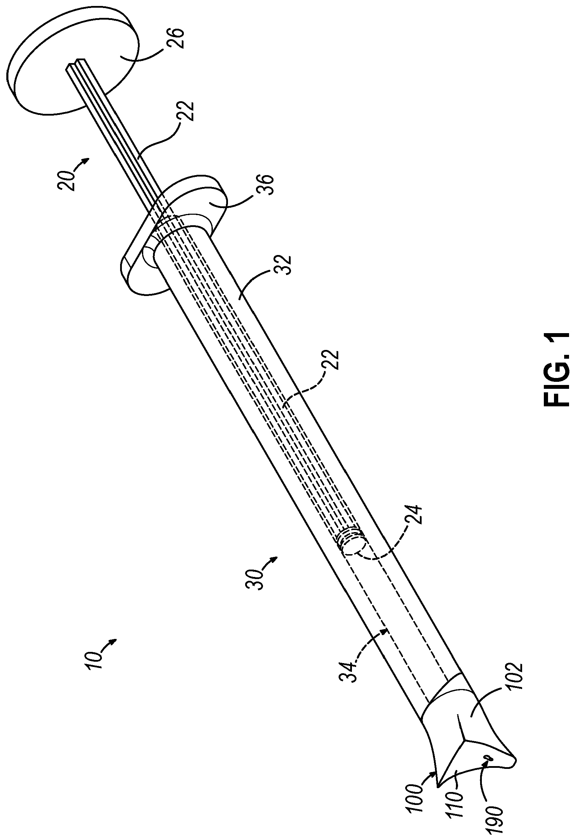

FIGS. 1-7 depict one example of an instrument (10) that may be used to deliver a therapeutic agent to a target location in an eye of a patient. Instrument (10) of this example includes a plunger (20) that is slidably disposed with respect to a body (30). Plunger (20) includes a shaft (22). A piston (24) is located at a distal end of shaft (22) while a plunger (26) is located at the proximal end of shaft (22). Body (30) includes a barrel (32) defining a hollow interior region (34) and a hilt (36). Piston (24) is slidably positioned in hollow interior region (34) such that a liquid may be contained in the portion of interior region (34) that is distal to piston (24); and such that the liquid may be dispensed from interior region (34) by distal advancement of piston (24) relative to barrel (32). Plunger (20) and body (30) may thus be operated like a syringe, with piston (24) and hollow interior region (34) cooperating to define a fluid reservoir.

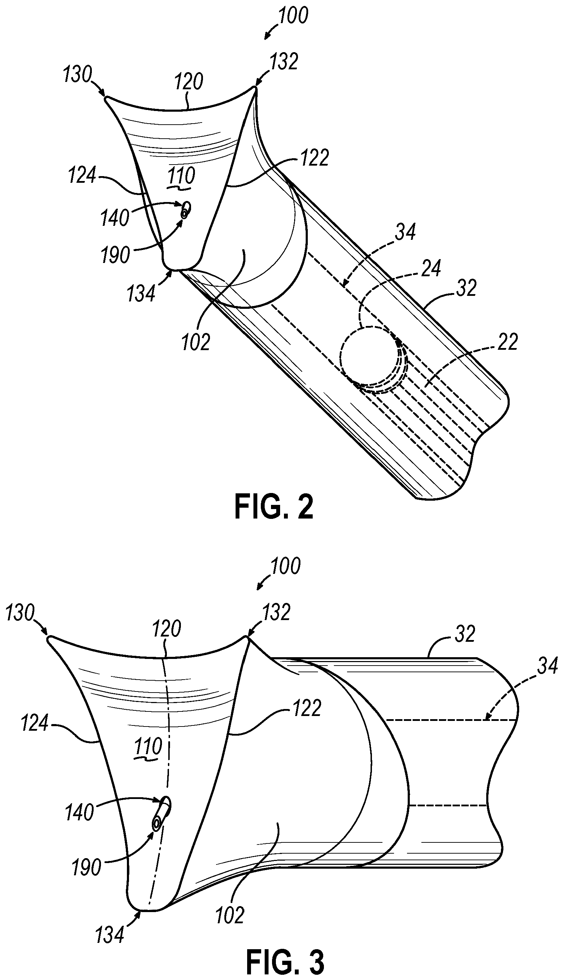

Instrument (10) of the present example further includes a head (100) at the distal end of barrel (32). In some versions, head (100) may be integrally formed with barrel (32). In some other versions, head (100) may be secured to barrel (32) via a conventional luer fitting, via some other kind of threaded fitting, via a snap fitting, or in any other suitable fashion as will be apparent to those skilled in the art in view of the teachings herein. As best seen in FIGS. 2-5, head (100) of the present example includes a body (102) with a distal face (110) that is bounded by three distal edges (120, 122, 124) with three corners (130, 132, 134). Each distal edge (120, 122, 124) has a curvature that is concave along a longitudinal dimension and along a lateral dimension. In this context, the term "longitudinal dimension" refers to the dimension along which the central longitudinal axis of body (30) extends; while "lateral dimension" refers to a dimension that is perpendicular to the central longitudinal axis of body (30). Distal face (110) also has a concave curvature along the longitudinal and lateral dimensions. As described in greater detail below, the curvature of upper distal edge (120) is configured to complement the curvature of a limbus (L) of a patient's eye (E); while the curvature of distal face (110) is configured to complement the curvature of the exterior surface of the patient's eye (E).

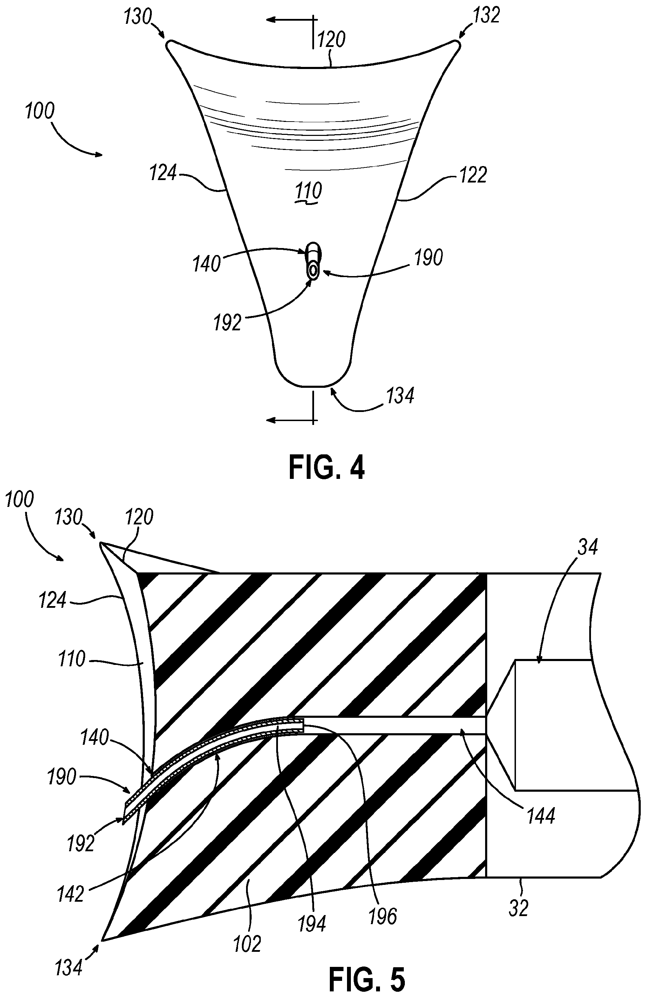

As best seen in FIG. 4, corners (130, 132, 134) are generally rounded such that corners (130, 132, 134) are atraumatic. Thus, when distal face (110) is urged against a patient's eye (E) as described in greater detail below, corners (130, 132, 134) will not inflict trauma on the patient's eye (E).

A needle (190) protrudes distally from distal face (110) in this example. As best seen in FIG. 5, needle (190) is disposed in an internal passageway (140) formed in head (100). A sharp distal tip (192) of needle (190) protrudes distally relative to distal face (110). The sharpness of distal tip (192) is provided by a bevel in this example. By way of example only, the angle of the bevel at distal tip may range from approximately 5 degrees to approximately 45 degrees; or may be approximately 15 degrees. In some versions, this bevel angle of distal tip (192) may complement the angle of the layers of tissue (S, Ch, R) in the eye (E) relative to the longitudinal axis of needle (190), such that distal tip (192) remains parallel with the boundaries of those layers as needle (190) is inserted into the eye (E). Such a bevel angle may prevent or minimize intrusion of distal tip (192) into the choroid (Ch). In some other versions, the bevel angle of distal tip (192) may be selected such that distal tip (192) is obliquely oriented relative to the boundaries of the layers of tissue (S, Ch, R) in the eye (E). Such a bevel angle may increase the likelihood of therapeutic agent reaching the suprachoroidal space (SCS) in the event that a portion of distal tip (192) remains in the sclera (S) and/or a portion of distal tip (192) overshoots the suprachoroidal space (SCS) and is disposed in the choroid (Ch).

A proximal end (196) of needle (190) is positioned in passageway (140), with a proximal portion (144) of passageway (140) extending proximally from proximal end (196) of needle (190) to hollow interior region (34) of barrel (32). A lumen (194) of needle (190) is in fluid communication with proximal portion (144) of passageway (140), such that fluid (e.g., including therapeutic agent) contained within hollow interior region (34) of barrel (32) may be expelled out through distal tip (192) of needle (190). In the present example, needle (190) is fixedly secured within passageway (140), such that needle (190) does not translate longitudinally within passageway (140). In some other versions, examples of which are described in greater detail below, needle (190) is operable to translate longitudinally within passageway (140) (e.g., in response to an operator input).

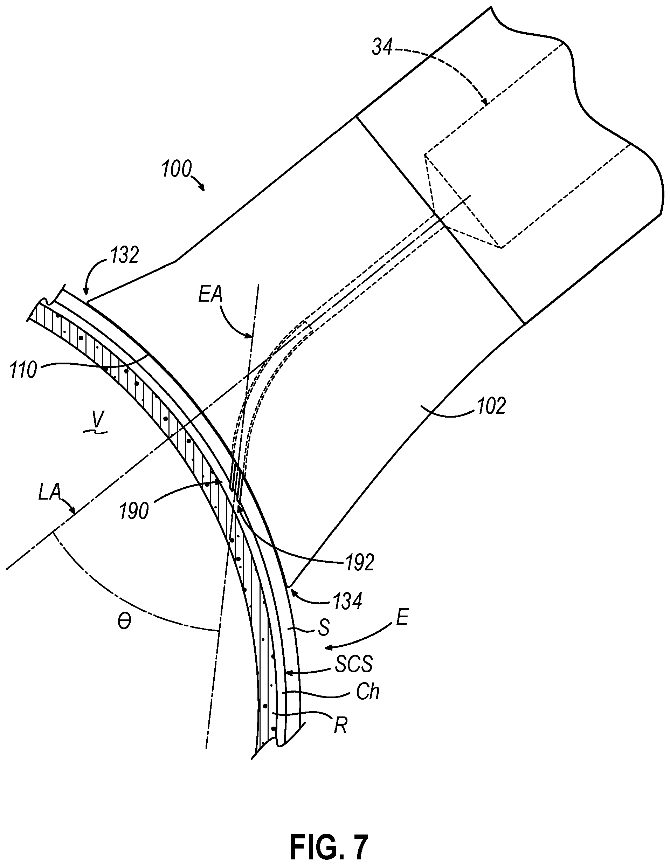

In the present example, proximal portion (144) of passageway (140) is straight, such that proximal portion (144) of passageway (140) extends along the longitudinal axis (LA) of head (100) (FIG. 7). In the present example, the longitudinal axis (LA) of head (100) is coaxially aligned with the longitudinal axis of body (30). A distal portion (142) of passageway (140) extends along a curve. With needle (190) being disposed in distal portion (142) of passageway (140), this curve of distal portion (142) imparts a corresponding curve to needle (190). As shown in FIG. 7 and as will be described in greater detail below, this curve of distal portion (142) and needle (190) provides an exit axis (EA) for needle (190) that is obliquely oriented relative to the longitudinal axis (LA). In this context, the exit axis (EA) is the axis along which distal tip (192) is oriented. The angle (Θ) defined between the exit axis (EA) of needle (190) and the longitudinal axis (LA) may be referred to as an exit angle. By way of example only, the exit angle (Θ) may range from approximately 50 degrees to approximately 89 degrees; or may be approximately 85 degrees. While the exit angle (Θ) is fixed in the present example, other versions may allow the operator to selectively vary the exit angle (Θ), as will be described in greater detail below. In some scenarios, the longitudinal axis (LA) passes through the center of the eye (E) when distal face (110) is fully seated on the eye (E).

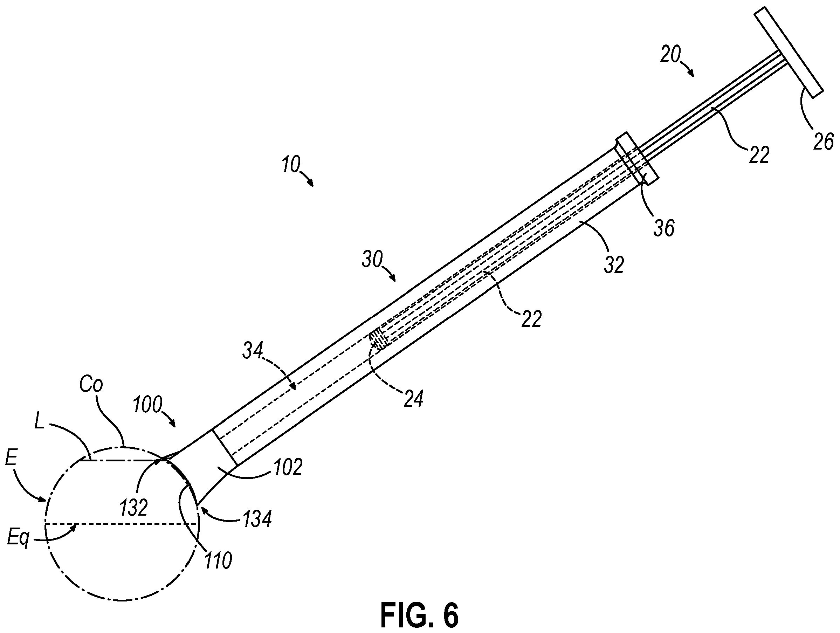

FIGS. 6-7 show an example of instrument (10) in use. As shown in FIG. 6, instrument (10) is positioned such that distal face (110) abuts the outer surface (i.e., conjunctiva) of a patient's eye (E). The operator positions and orients head (100) such that upper distal edge (120) is positioned along the outer curvature of the limbus (L) of the patient's eye (E), with lower corner (134) being oriented toward the equator (Eq) of the patient's eye (E). By indexing off of the limbus (L), upper distal edge (120) may be used to ensure that needle (190) enters the eye (E) at the appropriate location between the limbus (L) and the equator (Eq) on a reliable, consistent basis. In other words, with upper distal edge (120) serving as a guide, the operator does not need to utilize calipers or other instrumentation in order to identify and mark the appropriate needle (190) insertion location relative to the limbus (L). With upper distal edge (120) indexed along the limbus (L), the operator may urge head (100) against the eye (E) until distal face (110) is fully seated against the eye (E).

As distal face (110) is fully seated against the eye (E), as shown in FIG. 7, needle (190) penetrates the sclera (S) of the eye (E) and at least a portion of distal tip (192) is positioned in the suprachoroidal space (SCS). With at least a portion of distal tip (192) positioned in the suprachoroidal space (SCS), the operator may actuate plunger (20) distally relative to body (30), thereby expelling the therapeutic agent contained in hollow interior region (34) of barrel (32) into the suprachoroidal space (SCS) via needle (190).

In some versions, at least a portion of distal tip (192) reaches the choroid (Ch) (e.g., contacting the choroid (Ch) without necessarily piercing the choroid (Ch)) when distal face (110) is fully seated against the eye (E). In such scenarios, the therapeutic agent contained in hollow interior region (34) of barrel (32) may nevertheless still reach the suprachoroidal space (SCS) when the operator actuates plunger (20) distally relative to body (30). The bevel angle of distal tip (192) may assist in directing fluid that is expelled from needle (190) into the suprachoroidal space (SCS), even if a portion of distal tip (192) is in contact with the choroid (Ch) at the time the fluid is expelled. Moreover, the exit angle (Θ) of needle (190), as provided by distal portion (142) of passageway (140) of head (100), is also configured to promote distal tip (192) reaching the suprachoroidal space (SCS) when distal face (110) is fully seated against the eye (E). The length of the portion of needle (190) that is exposed relative to distal face (110) also provides positioning of distal tip (192) in the suprachoroidal space (SCS) when distal face (110) is fully seated against the eye (E). Thus, the successful delivery of therapeutic agent to the suprachoroidal space (SCS) is influenced by a combination of the bevel angle of distal tip (192), the exit angle (Θ) of needle (190), and length of the portion of needle (190) that is exposed relative to distal face (110).

In the present example, distal tip (192) never penetrates the retina (R) and never reaches the vitreous chamber (V). Thus, distal tip (192) penetrates no further than the choroid (Ch). In some other variations, at least a portion of distal tip (192) penetrates the retina (R). Moreover, distal tip (192) may reach the vitreous chamber (V) in some variations.

In the procedure described above, the fluid is delivered to the anterior region of the eye (E). In some scenarios, after the fluid is delivered to the anterior region of the eye (E), at least some of the fluid (e.g., including a therapeutic agent) may eventually disperse through the suprachoroidal space (SCS) toward the posterior region of the eye (E). This may be beneficial when treating ocular conditions associated with the posterior region of the eye (E) (e.g., macular degeneration, etc.).

While plunger (20) is described herein as being utilized to drive fluid from hollow interior region (34) out through needle (190), any other suitable kind of fluid driving feature(s) may be used in addition to, or in lieu of, plunger (20) to serve such purposes.

### B. Example of Instrument with Translating Needle and Sensor

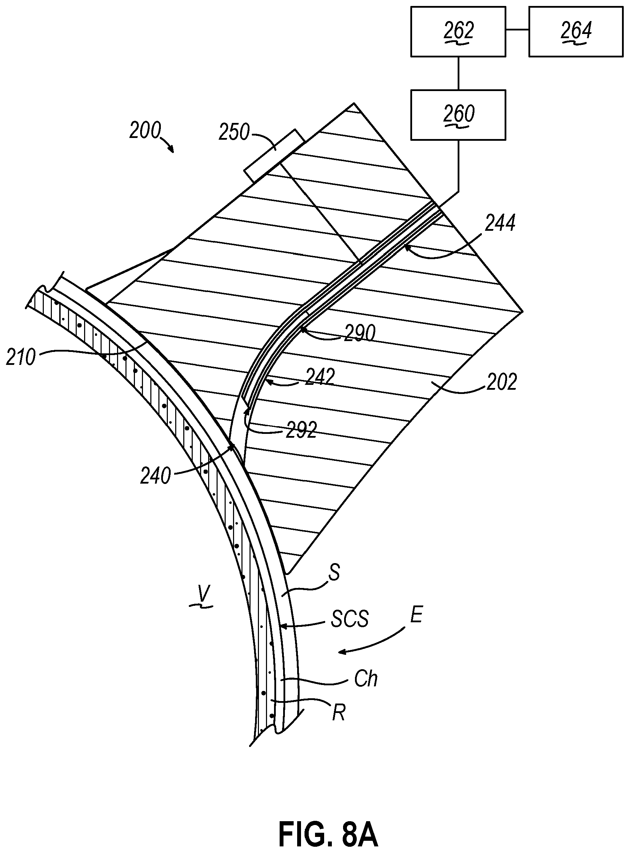

In some instances, it may be desirable to provide a variation of instrument (10) where needle (190) is contained within head (100) until distal face (110) is fully seated against the eye (E), such that needle (190) will only be advanced distally through the sclera (S) after distal face (110) is fully seated against the eye (E). To that end, FIGS. 8A-8B depicts an alternative example of a head (200) that may be integrated into instrument (10) as a substitute for head (100). Head (200) of this example may be configured and operable just like head (100) except for the differences described below. Head (200) of this example includes a distal face (210) that is configured just like distal face (110); and a passageway (240) extending from interior region (34) of barrel (32) to distal face (210). Head (200) of this example also includes a needle (290) with a sharp distal tip (292).

Unlike needle (190) of head (100), needle (290) of head (200) is configured to translate longitudinally relative to passageway (240). To provide such translation, needle (290) is coupled with an actuator (250). By way of example only, actuator (250) may include a slider, a dial, a knob, a lever, or any other suitable kind of actuator as will be apparent to those skilled in the art in view of the teachings herein. While actuator (250) is shown as being integrated into head (200), actuator (250) may instead be positioned at any other suitable location, including but not limited to body (30), etc. Regardless of the location of actuator (250), actuator (250) may be coupled with needle (250) in any suitable fashion as will be apparent to those skilled in the art in view of the teachings herein.

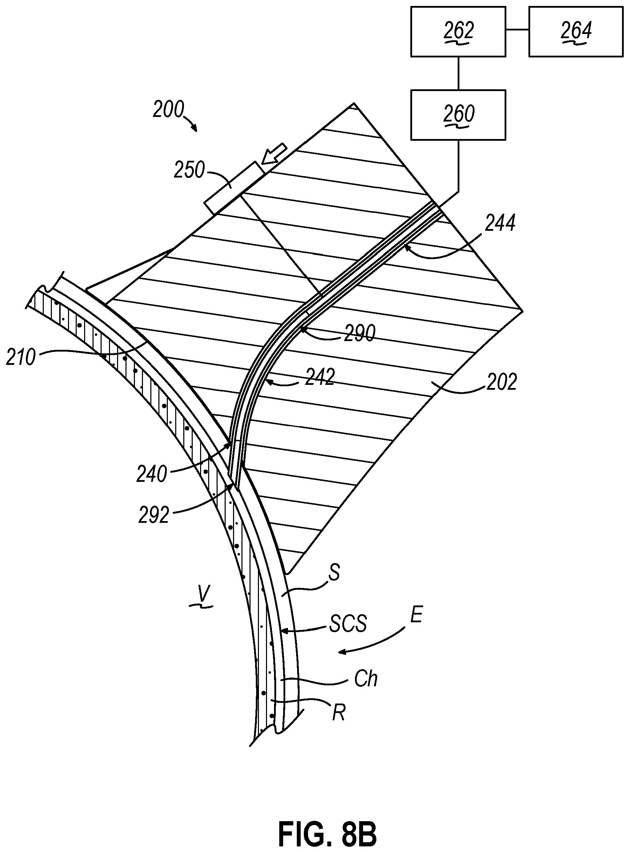

When actuator (250) is in a pre-actuated state as shown in FIG. 8A, distal tip (292) of needle (290) is positioned proximally in relation to distal face (210). This retraction of needle (290) into head (200) may help protect distal tip (292) from inadvertent damage; and may protect the operator and patient from inadvertent trauma that might otherwise be provided by distal tip (292). Head (200) may remain in this state up until the point at which distal face (210) is fully seated against the eye (E) as shown in FIG. 8A. Once distal face (210) is fully seated against the eye (E), the operator may manipulate actuator (250) to reach an actuated state as shown in FIG. 8B. In this actuated state, needle (290) has penetrated the sclera (S) and distal tip (292) is positioned in the suprachoroidal space (SCS). With distal tip (292) positioned in the suprachoroidal space (SCS), the operator may actuate plunger (20) distally relative to body (30), thereby expelling the therapeutic agent contained in hollow interior region (34) of barrel (32) into the suprachoroidal space (SCS) via needle (290) as described above.

During the transition from the state shown in FIG. 8A to the state shown in FIG. 8B, the curved distal portion (242) of passageway (242) may guide needle (290) to achieve a desired exit angle (Θ), as also described above. In addition to curved distal portion (242) of passageway (242) guiding needle (290) to achieve a desired exit angle (Θ), or as an alternative to curved distal portion (242) of passageway (242) guiding needle (290) to achieve a desired exit angle (Θ), needle (290) may include a resilient pre-formed bend that assists needle (290) in achieving the desired exit angle (Θ) as the distal portion of needle (290) is exposed relative to distal face (210) in the actuated state.

As with needle (190) of head (100), the successful delivery of therapeutic agent to the suprachoroidal space (SCS) via needle (290) of head (200) is influenced by a combination of the bevel angle of distal tip (292), the exit angle (Θ) of needle (290), and length of the portion of needle (290) that is exposed relative to distal face (210). In some versions, actuator (250) is configured to arrest distal advancement of needle (290) at a point where actuator (250) will prevent distal tip (292) from penetrating the retina (R) and/or reaching the vitreous chamber (V). In some versions where actuator (250) is operated manually, actuator (250) may provide the operator with tactile feedback that will enable the operator to feel when distal tip (292) has reached the suprachoroidal space (SCS), such that the operator may arrest actuation of actuator (250) once the operator feels (via actuator (250)) distal tip (292) reaching the suprachoroidal space (SCS). For instance, as distal tip (292) traverses the sclera (S), the operator may feel substantial resistance from the relatively tough tissue of the sclera (S); but then a sudden reduction in resistance once distal tip (292) has fully penetrated the sclera (S) and reached the suprachoroidal space (SCS). Some versions may provide a mechanism that amplifies the tactile feedback felt through actuator (250) as distal tip (292) traverses the sclera (S) and ultimately reaches the suprachoroidal space (SCS). Such tactile feedback amplification mechanisms may include one or more gears and/or other components as will be apparent to those skilled in the art in view of the teachings herein.

In addition to, or as an alternative to, providing tactile feedback through actuator (250), a version of instrument (10) with head (200) may include one or more components that are operable to sense the depth position of needle (290) within the eye (E), to thereby determine when distal tip (292) has reached the suprachoroidal space (SCS). To that end, in the example shown in FIGS. 8A-8B, a strain sensor (260) is in communication with needle (290). Strain sensor (260) is operable to sense the strain on needle (290) as needle is advanced distally from the position shown in FIG. 8A to the position shown in FIG. 8B. Strain sensor (260) is thus operable to generate a signal that is indicative of when distal tip (292) has reached the suprachoroidal space (SCS), as the strain in needle (290) will suddenly drop once distal tip (292) reaches the suprachoroidal space (292). Various suitable forms that strain sensor (260) may take will be apparent to those skilled in the art in view of the teachings herein.

In the present example, strain sensor (260) is in communication with a processing module (262), which is also in communication with a response module (264). Processing module (262) is operable to process signals from strain sensor (260) and drive response module (264) based on the signals from strain sensor (260). By way of example only, processing module (262) may include a microprocessor, an application specific integrated circuit (ASIC), and/or any other suitable components as will be apparent to those skilled in the art in view of the teachings herein. Response module (264) is configured to provide one or more responses in response to a command signal that is issued by processing module (262) based on strain sensor (260) indicating that distal tip (292) has reached the suprachoroidal space (SCS). Strain sensor (260), processing module (262), and response module (264) may positioned at any suitable location(s) within instrument (10), including but not limited to head (200) and/or body (30).

In some versions, response module (264) is operable to provide user feedback to the operator to indicate that distal tip (292) has reached the suprachoroidal space (SCS). For instance, response module (264) may include a tactile feedback feature that provides haptic feedback (e.g., a vibration, etc.) to the operator via actuator (250) and/or via body (30), etc., to indicate that distal tip (292) has reached the suprachoroidal space (SCS). In addition, or in the alternative, response module (264) may illuminate a light or provide some other form of visual feedback to indicate that distal tip (292) has reached the suprachoroidal space (SCS). In addition, or in the alternative, response module (264) may emit an audible tone or provide some other form of audible feedback to indicate that distal tip (292) has reached the suprachoroidal space (SCS). Various suitable components that may be integrated into response module (264) to provide tactile, visual, and/or audible feedback to the operator to indicate that distal tip (292) has reached the suprachoroidal space (SCS) will be apparent to those skilled in the art in view of the teachings herein.

In addition to providing user feedback, or in the alternative to providing user feedback, response module (264) may affect communication of fluid from interior region (34) of barrel (32) to needle (290) based on whether distal tip (292) has reached the suprachoroidal space (SCS). For instance, response module (264) may include a valve interposed between interior region (34) of barrel (32) and needle (290), with the valve remaining in a closed state until a strain sensor (260) detects distal tip (292) reaching the suprachoroidal space (SCS). With the valve in the closed state, fluid may not be communicated from interior region (34) of barrel (32) to needle (290). Once strain sensor (260) detects that distal tip (292) has reached the suprachoroidal space (SCS), processing module (262) may send a command signal to response module (264) to open the valve, thereby enabling fluid to be communicated from interior region (34) of barrel (32) to needle (290). In some versions having a valve as part of response module (264), the valve may provide misalignment between a fluid channel from interior region (34) of barrel (32) leading to needle (290), such that the misalignment provides a closed state for the valve. Once strain sensor (260) detects distal tip (292) reaching the suprachoroidal space (SCS), processing module (262) may activate the valve of response module (264) to provide alignment between the fluid channel from interior region (34) of barrel (32) and needle (290), such that the alignment provides an open state for the valve.

In addition, or in the alternative, response module (264) may include a pump or other feature that is operable to actively drive fluid from interior region (34) of barrel (32) to needle (290) in response to a signal indicating that distal tip (292) has reached the suprachoroidal space (SCS). In such versions, plunger (20) may be omitted. Various suitable components that may be integrated into response module (264) to provide the above-describe fluid communication effects will be apparent to those skilled in the art in view of the teachings herein. Similarly, other suitable kinds of responses that may be carried out by response module (264), and components that may be incorporated into response module (264) for carrying out such other responses, will be apparent to those skilled in the art in view of the teachings herein.

While strain sensor (260), processing module (262), and response module (264) are described above in the context of head (200), such components are optional and may be omitted in some versions of instrument (10) that include head (200). Similarly, the other variations of instrument (10) that are described herein, including but not limited to the variations including head (100, 300, 400, 500, 700) may include strain sensor (260), processing module (262), and response module (264), if desired.

### C. Example of Instrument with Translating Pre-Curved Needle

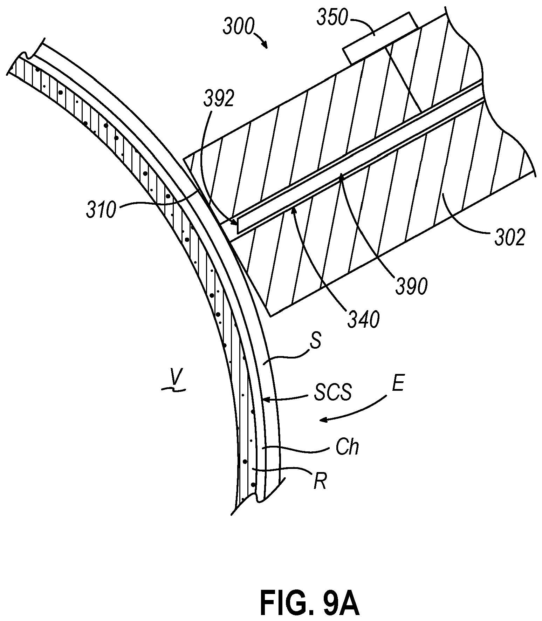

In the above-described examples of heads (100, 200), the obliquely oriented exit axis (EA) of needle (190, 290) is provided by the curved distal portion (142, 242) of passageway (140, 240). In such versions, needle (190, 290) may in fact be resiliently biased to assume a straight configuration, with the curved distal portion (142, 242) of passageway (140, 240) imparting lateral stress on needle (190, 290) in order to deform needle (190, 290) to achieve the oblique exit angle (Θ) from distal face (110, 210) of head (100, 200). In some other variations, the needle itself may be resiliently biased to have a bent distal end, such that the needle is pre-curved. An example of such a variation is shown in FIGS. 9A-9B.

FIGS. 9A-9B show an alternative example of a head (300) that may be integrated into instrument (10) as a substitute for head (100). Head (300) of this example may be configured and operable just like head (100) except for the differences described below. Head (300) of this example includes a distal face (310) and a passageway (340) extending from interior region (34) of barrel (32) to distal face (310). Head (300) of this example also includes a needle (390) with a sharp distal tip (392). Needle (390) is configured to translate longitudinally relative to passageway (340). To provide such translation, needle (390) is coupled with an actuator (350). By way of example only, actuator (350) may be configured and operable like actuator (250) described above. Other suitable configurations and operabilities for actuator (350) will be apparent to those skilled in the art in view of the teachings herein.

In the present example, distal face (310) is substantially flat, such that distal face (310) is not contoured to complement the curvature of the eye (E). In some other versions, distal face (310) is in fact contoured to complement the curvature of the eye (E). Distal face (310) may thus be configured and operable similar to distal face (110, 210) of head (110, 210). In versions where distal face (310) is flat, the operator may pivot head (300) at the interface between distal face (310) and the eye (E) to effectively adjust the angle at which needle (390) enters the eye (E). Regardless of whether distal face (310) is flat or contoured, distal face (310) may include an upper distal edge or other guidance feature that is configured to facilitate indexing of head (300) relative to the limbus (L) of the eye (E) to thereby ensure that needle (390) enters the eye at the desired position between the limbus (L) and the equator (Eq).

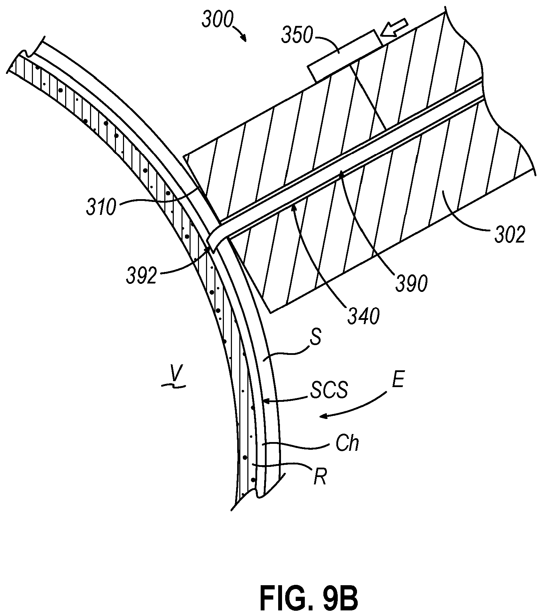

The entire length of passageway (340) is straight in this example. In some versions, the entire length of passageway (340) is coaxial with the central longitudinal axis of head (300) and/or body (30). In some versions where the entire length of passageway (340) is straight, the resilient bias in the distal portion (394) of needle (390) is enough to achieve the desired oblique exit axis (EA) as distal portion (394) of needle (390) exits distal face (310) (FIG. 9B). In some other versions, a distal portion of passageway (340) is curved or otherwise bent. For instance, a distal portion of passageway (340) may be configured similar to distal portion (142, 242) of passageway (140, 240). In such versions, the resilient bias in the distal portion (394) of needle (390), together with deformation induced by a curved distal portion of passageway (340), may provide the desired oblique exit axis (EA) as distal portion (394) of needle (390) exits distal face (310).

When actuator (350) is in a pre-actuated state as shown in FIG. 9A, distal tip (392) of needle (390) is positioned proximally in relation to distal face (310). This retraction of needle (390) into head (300) may help protect distal tip (392) from inadvertent damage; and may protect the operator and patient from inadvertent trauma that might otherwise be provided by distal tip (392). With passageway (340) being straight in this example, and with distal portion (394) being resiliently biased to achieve a curved configuration, passageway (340) may hold distal portion (394) in a stressed state while actuator (350) is in a pre-actuated state as shown in FIG. 9A. Head (300) may remain in this state up until the point at which distal face (310) is placed in contact with the eye (E) as shown in FIG. 9A.

Once distal face (310) is in sufficient contact with the eye (E), the operator may manipulate actuator (350) to reach an actuated state as shown in FIG. 9B. During such actuation, as the distal portion (394) exits distal face (310), the resilient bias of distal portion (394) will cause the length of distal portion (394) that is exposed relative to distal face (310) to bend, thereby causing distal tip (392) to deflect laterally along an obliquely oriented exit axis (EA). During this actuation, needle (390) penetrates the sclera (S) and distal tip (392) is eventually positioned in the suprachoroidal space (SCS). In some versions, the pre-formed bend in distal portion (394) of needle (390) may be carefully configured to promote the bevel angle of distal tip (392) remaining substantially parallel with the boundaries of the layers of tissue (S, Ch, R) in the eye (E). Regardless of the configuration of distal tip (392), the pre-formed bend in distal portion (394) of needle (390) may be carefully configured to promote entry of distal tip (392) into the suprachoroidal space (SCS) at an optimal angle. With distal tip (392) positioned in the suprachoroidal space (SCS), the operator may actuate plunger (20) distally relative to body (30), thereby expelling the therapeutic agent contained in hollow interior region (34) of barrel (32) into the suprachoroidal space (SCS) via needle (390) as described above.

In some versions of head (300), needle (390) is formed of nitinol. Alternatively, any other suitable material or combination of materials may be used to form needle (390). Other suitable material(s) that may be used to form needle (390) will be apparent to those skilled in the art in view of the teachings herein.

### D. Example of Instrument with Adjustable Angle Needle

In the examples described above, a curved distal portion (142, 242) of passageway (140, 240) and/or a pre-formed bend in distal portion (394) of needle (390) is used to achieve a desired exit angle (Θ) for needle (190, 290, 390). In the example of heads (100, 200), the curvature of curved distal portion (142, 242) of passageway (140, 240) may be fixed, such that the operator may be unable to make adjustments to the exit angle (Θ) if such adjustments are necessary to reach the suprachoroidal space (SCS) of a particular patient at hand. Similarly, in the example of head (300), the curvature of the pre-formed bend in distal portion (394) of needle (390) may be fixed, such that the operator may be unable to make adjustments to the exit angle (Θ) if such adjustments are necessary to reach the suprachoroidal space (SCS) of a particular patient at hand. It may therefore be desirable to provide a variation of instrument (10) that allows the operator to adjust the exit angle (Θ) of a needle if such adjustments are necessary to reach the suprachoroidal space (SCS) of a particular patient at hand.

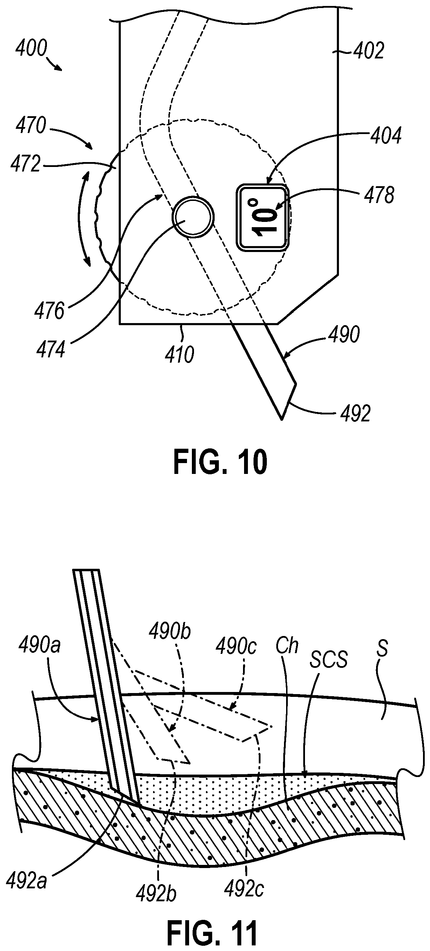

FIGS. 10 depicts an alternative example of a head (400) that may be integrated into instrument (10) as a substitute for head (100). Head (400) of this example may be configured and operable just like head (100) except for the differences described below. Head (400) of this example includes a distal face (410) and an adjustment member (470) rotatably supported by a body (402) of head (400). Distal face (410) is shown as being flat in the present example, though other versions of distal face (410) may be contoured to complement the curvature of the eye (E) (e.g., similar to distal face (110, 210). As will be described in greater detail below, a needle (490) passes through adjustment member (470), with adjustment member (470) being operable to adjust the exit angle (Θ) of needle (490). In some versions, the longitudinal position of needle (490) is fixed relative to the body (402) of head (400) (e.g., similar to the relationship between needle (190) and body (102) of head (100)). In some other versions, an actuator (e.g., similar to actuator (290, 390)) is operable to drive longitudinal translation of needle (490) relative to body (402) of head (400).

Adjustment member (470) includes a wheel (472) with an axle (474). Wheel (472) is pivotably coupled with body (402) via axle (474) such that wheel (472) is rotatable relative to body (402) about the rotation axis defined by axle (474). A portion of wheel (472) is exposed relative to body (402) to thereby enable an operator to engage wheel (472) with a finger or thumb and thereby rotate wheel (472) relative to body (402). Wheel (472) further defines a passageway (476). A needle (490) is disposed in passageway (476). In the present example, passageway (476) is positioned such that passageway (476) perpendicularly intersects the rotation axis defined by axle (474). In other versions, passageway (476) may be offset in relation to the rotation axis defined by axle (474) such that the rotation axis defined by axle (474) does not intersect passageway (476).

As shown in FIG. 11, when the operator rotates wheel (472) relative to body (402), the operator may effectively reposition needle (490) at various exit angles (Θ), thereby achieving a variety of alternative use positions (490a, 490b, 490c). In alternative use position (490c), distal tip (492c) is positioned entirely in the sclera (S), such that therapeutic agent expelled via distal tip (492c) may not necessarily reach the suprachoroidal space (SCS). In alternative use position (490b), distal tip (492b) is closer to reaching the suprachoroidal space (SCS), but is still positioned entirely in the sclera (S), such that therapeutic agent expelled via distal tip (492b) may again not necessarily reach the suprachoroidal space (SCS). However, in alternative use position (490a), distal tip (492a) is within the suprachoroidal space (SCS), such that therapeutic agent expelled via distal tip (492a) will effectively reach the suprachoroidal space (SCS).

During use of an instrument (10) incorporating head (400), the operator may advance distal face (410) into engagement with the eye (410), with adjustment member (470) set to provide a first exit angle (Θ) for needle (490). In versions where the longitudinal position of needle (490) is fixed relative to body (402), the operator may advance distal tip (492) into the eye (E) simply by urging distal face (410) into contact with the eye (E). In versions where an actuator is used to longitudinally translate needle (490) relative to body (402), the operator may first bring distal face (410) into contact with the eye (E), then operate the actuator to advance distal tip (492) into the eye (E). In either case, the operator may rely on any suitable form of feedback to determine whether the advanced distal tip (492) successfully reached the suprachoroidal space (SCS). By way of example only, the operator may rely on tactile feedback via the hand grasping instrument (10). Alternatively, the operator may rely on feedback from another feature like response module (264). Other suitable ways in which the operator may determine whether the advanced distal tip (492) successfully reached the suprachoroidal space (SCS) will be apparent to those skilled in the art in view of the teachings herein.

If the operator determines that the advanced distal tip (492) has not successfully reached the suprachoroidal space (SCS), the operator may retract needle (490) from the eye (E) and then manipulate wheel (472) to adjust the exit angle (Θ). To assist in this adjustment process, wheel (472) of the present example includes indicia (478) and body (478) defines a window (404) through which indicia (478) may be visually observed. Indicia (478) may indicate the exit angle (Θ) associated with the current angular position of wheel (472). Wheel (472) and body (402) may further include detents or other features that assist in releasably maintaining wheel (472) at certain discrete angular positions associated with corresponding exit angles (Θ). Once the operator has adjusted the exit angle (Θ) by manipulating wheel (472), the operator may again advance distal tip (492) into the eye (E) at the adjusted exit angle (Θ). The above-described process may be repeated until the operator determines that distal tip (492) has successfully reached the suprachoroidal space (SCS). Once the operator determines that distal tip (492) has successfully reached the suprachoroidal space (SCS), the operator may administer the therapeutic agent to the suprachoroidal space (SCS) via the needle as described above.

While a wheel (472) is described above as an example of a structure that may be used to provide adjustability of the exit angle (Θ) of a needle (490), any other suitable kind of structures may be used. Other suitable structures that may be used to provide adjustability of the exit angle (Θ) of a needle (490) will be apparent to those skilled in the art in view of the teachings herein. While adjustment member (470) is described herein in the context of head (400), adjustment member (470) or variations thereof may be readily incorporated into any of the other various heads (100, 200, 300, 500, 800) described herein.

### E. Example of Instrument with Voltage Sensing

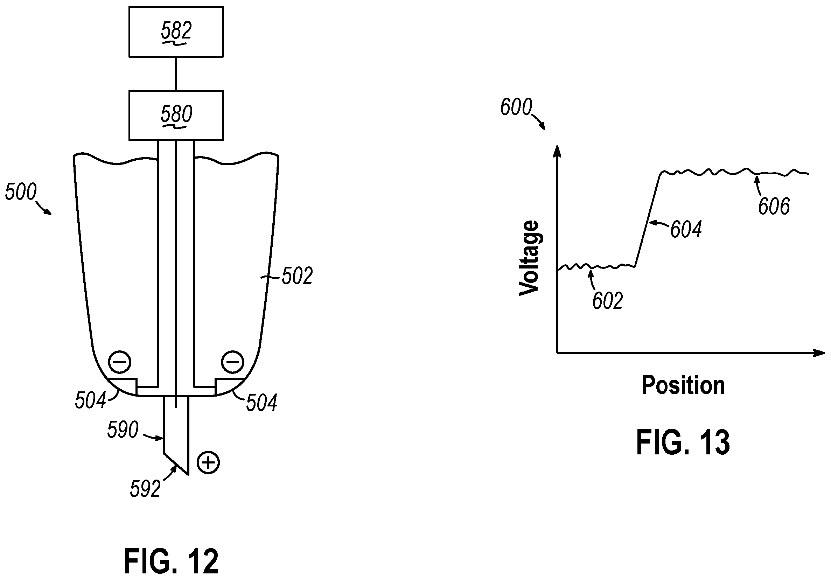

As described above in the context of head (200), a strain sensor (260) may be used to detect the position of needle (290) within the layers of tissue (S, Ch, R) in the eye (E) as a function of the stress encountered by needle (290) during advancement of needle (290) into the eye (E). FIG. 12 depicts another example of how an instrument may detect the position of needle (290) within the layers of tissue (S, Ch, R) in the eye (E). In particular, FIG. 12 depicts a head (500) that may be integrated into instrument (10) as a substitute for head (100). Head (500) of this example may be configured and operable just like head (100) except for the differences described below. Head (500) of this example includes a needle (590), a set of distal electrodes (504), a processing module (580), and a response module (582). Needle (590) is formed of an electrically conductive material such that needle (590) is operable to serve as an electrode (e.g., at a different polarity from electrodes (504)). Processing module (580) is in electrical communication with needle (590) and electrodes (504).

Electrodes (504) are positioned and configured to contact tissue of the eye (E) as head (500) is urged into contact with the eye (E). In some versions, electrodes (504) include one or more electrodes (504) that are spaced apart from each other in an angularly spaced array about the longitudinal axis of body (502) or needle (590). In some other versions, electrodes (504) are provided in the form of one or more annular electrodes that is/are coaxially positioned about the longitudinal axis of body (502) or needle (590). Other suitable forms that electrodes (504) may take will be apparent to those skilled in the art in view of the teachings herein.

When needle (590) and electrodes (504) are simultaneously in contact with tissue, processing module (580) may detect an electrical potential or voltage in the tissue. FIG. 13 shows a plot (600) depicting an example of how such voltage may vary based on the position of distal tip (592) of needle (590) within the layers of tissue (S, Ch, R) in the eye (E). During initial insertion of needle (590) into the eye (E), a first portion (602) of the plot (600) shows a relatively low voltage as distal tip (592) traverses the sclera (S). As distal tip (592) reaches the suprachoroidal space (SCS), the plot (600) shows a sharp increase (604) in voltage, with the final portion (606) of plot (600) showing a relatively high voltage with distal tip (592) in the suprachoroidal space (SCS).

In some versions, needle (590) is configured to translate longitudinally relative body (502) such that distal tip (592) is retracted proximally in body (502) when distal electrodes (504) make initial contact with the eye (E). Once distal electrodes (504) are in contact with the eye (E), needle (590) may be advanced distally into the eye (E) as described in other examples herein, with processing module (580) tracking the voltage value as needle (590) is advanced distally.

When processing module (580) detects a voltage associated with distal tip (592) successfully reaching the suprachoroidal space (SCS) (e.g., consistent with the final portion (606) of plot (600) in FIG. 13), processing module (580) may activate response module (582) to provide a corresponding response. Response module (582) may be configured and operable like response module (264) described above. Thus, response module (582) may provide tactile, visual, and/or audible feedback to the operator to indicate that distal tip (592) has reached the suprachoroidal space (SCS); may open a valve, activate a pump, and/or provide other fluid communication effects in response to distal tip (592) reaching the suprachoroidal space (SCS); and/or provide any other suitable kind(s) of response(s) as will be apparent to those skilled in the art in view of the teachings herein.

While electrodes (504), needle (590), and processing module (580) are described herein as being used to track changes in voltage as a function of the depth of insertion of needle (590) into the eye (E), these components may instead be used to track any other suitable electrical parameter (e.g., impedance or electrical resistance, etc.) as a function of the depth of insertion of needle (590) into the eye (E), as will be apparent to those skilled in the art in view of the teachings herein. While electrodes (504), processing module (580), and response module (582) are described herein in the context of head (500), these components or variations thereof may be readily incorporated into any of the other various heads (100, 200, 300, 400, 800) described herein.

### F. Example of Instrument with Sclera Tensioning Feature

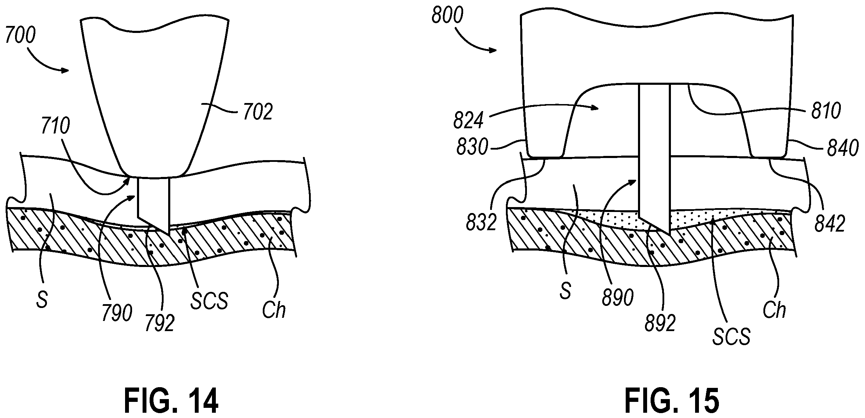

In some scenarios, an operator may inadvertently press the head of an instrument against an eye (E) with too much force, which may cause deformation in the layers of the eye (E). An example of such a scenario is shown in FIG. 14. As shown in FIG. 14, a head (700) of an instrument includes a needle (790) projecting distally from a body (702), with a distal face (710) being pressed into the eye (E) with substantial force. This pressure imposed by distal face (710) has caused the sclera (S) to deform and thereby press against the choroid (Ch), thereby compressing the suprachoroidal space (SC). While distal tip (792) of needle (790) may still be successfully positioned in the suprachoroidal space (SCS) and thereby delivery therapeutic agent to the suprachoroidal space (SCS), the compressed state of the suprachoroidal space (SCS) may adversely impact the ability of the therapeutic agent to be received and dispersed within the suprachoroidal space (SCS).

Thus, with a configuration of head (700) similar to that shown in FIG. 14, the success of the procedure may be substantially dependent on the skill of the operator, based on the extent to which the operator can avoid creating the conditions depicted in FIG. 14 by pressing head (700) into the eye (E) with too much force. It may therefore be desirable to provide an alternative configuration for head (700) that is less sensitive to the skill of the operator, such that the suprachoroidal space (SCS) is less likely to be compressed even if the operator presses the head into the eye (E) with substantial force. FIG. 15 depicts an example of such an alternative.

In particular, FIG. 15 depicts a head (800) that may be integrated into instrument (10) as a substitute for head (100). Head (800) of this example may be configured and operable just like head (100) except for the differences described below. Head (800) of this example includes a needle (890) extending distally from a distal surface (810), a first distal projection (830), and a second distal projection (840). Distal projections (830, 840) are laterally offset from needle (890). Each distal projection (830, 840) includes an eye engaging surface (832, 842). Distal surface (810) is located proximally in relation to eye engaging surfaces (832, 842). Thus, when head (800) is pressed against an eye (E) as shown in FIG. 15, eye engaging surfaces (832, 842) engage the sclera (S) while distal surface (810) remains recessed relative to the sclera (S), with a gap (824) being defined between distal surface (810) and the sclera (S). With eye engaging surfaces (832, 842) being laterally offset from needle (890), the pressure applied to the eye (E) by head (800) is laterally offset from the point at which needle (890) is inserted into the eye (E). Moreover, eye engaging surfaces (832, 842) maintain tension in the sclera (S); and the pressure imposed by head (800) on the eye (E) is distributed over a larger surface area of the eye (E) as compared to the pressure from head (700). Thus, the sclera (S) is less likely to deform in the region where needle (890) enters the sclera (S); and head (800) does not tend to compress the suprachoroidal space (SCS) in the region where distal tip (892) of needle (890) dispenses therapeutic agent to the suprachoroidal space (SCS). This may improve the ability of the therapeutic agent to be received and dispersed within the suprachoroidal space (SCS), as compared to the scenario with head (700) depicted in FIG. 14.

While head (800) of the present example is described as including two distal projections (830, 840), head (800) may include any other suitable number of distal projections (830, 840). For instance, head (800) may include three or more distal projections that are angularly spaced apart from each other about a central longitudinal axis of head (800) and/or needle (890). As another example, distal projections (830, 840) may be substituted or supplemented with a single distal projection that continuously spans a full circumference about needle (890), such that the single distal projection and distal surface (810) together define a cup-like shape. Other suitable configurations and variations will be apparent to those skilled in the art in view of the teachings herein. While distal projections (830, 840) are described herein in the context of head (800), distal projections (830, 840) or variations thereof may be readily incorporated into any of the other various heads (100, 200, 300, 400, 500) described herein.

### G. Example of Instrument with Fixed Position Needle and Flexible Conduit for Syringe Coupling

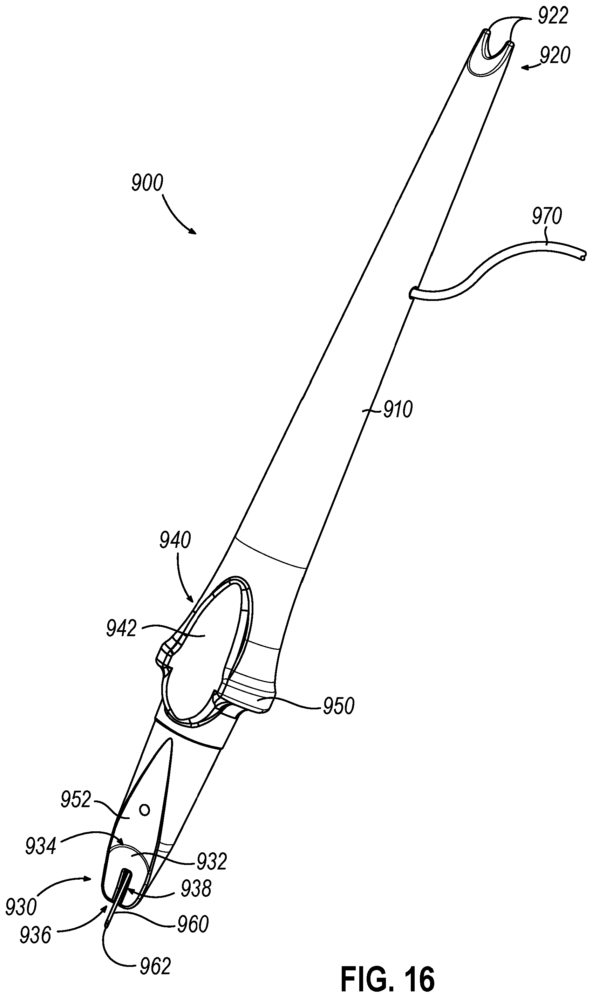

In some scenarios, it may be desirable to utilize a separate syringe to deliver fluid via an instrument to a suprachoroidal space (SCS), where the delivery instrument is coupled with the syringe via a flexible conduit. This may enable the operator to grasp the delivery instrument with one hand while grasping the syringe with another hand. Alternatively, this may enable a first operator to grasp the delivery instrument with one hand and another operator (e.g., an assistant) to grasp the syringe. In either scenario, it may be easier for the delivery instrument operator to maintain the position, orientation, and stabilization of the delivery instrument if the operator does not need to use the same hand that is grasping the delivery instrument to perform other functions (e.g., driving a plunger to deliver fluid, etc.). FIGS. 16-18C show an embodiment of a delivery instrument (900) according to the invention that may be grasped and manipulated by a single hand of an operator while another hand of the same operator (or the hand of another operator) grasps and operates a syringe (980).

As shown in FIG. 16, instrument (900) of this embodiment includes a shaft (910) that is configured for grasping with a single hand of the operator (e.g., via a pencil grip). A proximal end of shaft (910) includes an integral marking assembly (920), which includes a pair of proximally extending marking prongs (922) that are laterally spaced apart from each other. Prongs (922) of this example are blunt or otherwise atraumatic, such that prongs (922) may be pressed against a patient's eye (E) without piercing the sclera (S). The distal end of shaft (910) includes a head (930) that is used to engage an eye (E) of a patient to thereby deliver fluid (e.g., therapeutic agent, etc.) to the eye (E) as described herein. Head (930) includes a concave distal face (932). Distal face (932) is oriented generally transversely relative to the longitudinal axis of shaft (910). Distal face (932) is bounded by an upper edge region (934) and a lower edge region (936), with regions (934, 936) together defining an outer perimeter of distal face (932). Similar to distal face (110) of head (100) described above, distal face (932) of head (930) has a concave curvature along longitudinal and lateral dimensions. The curvature of distal face (932) is configured to complement the curvature of the exterior surface of the patient's eye (E).

Instrument (900) of the present embodiment further includes a laterally facing guidance feature (940) and a laterally extending grip feature (950) along an intermediate region of shaft (910). Laterally facing guidance feature (940) includes a concave face (942) having a curvature that is configured to complement the curvature of a limbus (L) of a patient's eye (E). As described in greater detail below, this configuration may enable concave face (942) to be used to visually facilitate alignment of instrument (900) relative to the eye (E). Grip feature (950) is configured to promote grasping of instrument (900) with a single hand, particularly using a pencil grip. Alternatively, instrument (900) may be grasped in any other suitable fashion. A laterally facing surface (952) extends between concave face (942) of laterally facing guidance feature (940) and upper edge region (934) of distal face (932). Surface (952) is obliquely oriented relative to the longitudinal axis of shaft (910) and has a concave curvature. Surface (952) is configured to avoid or minimize contact with the cornea of the eye (E) during use of instrument (900), as will be described in greater detail below.

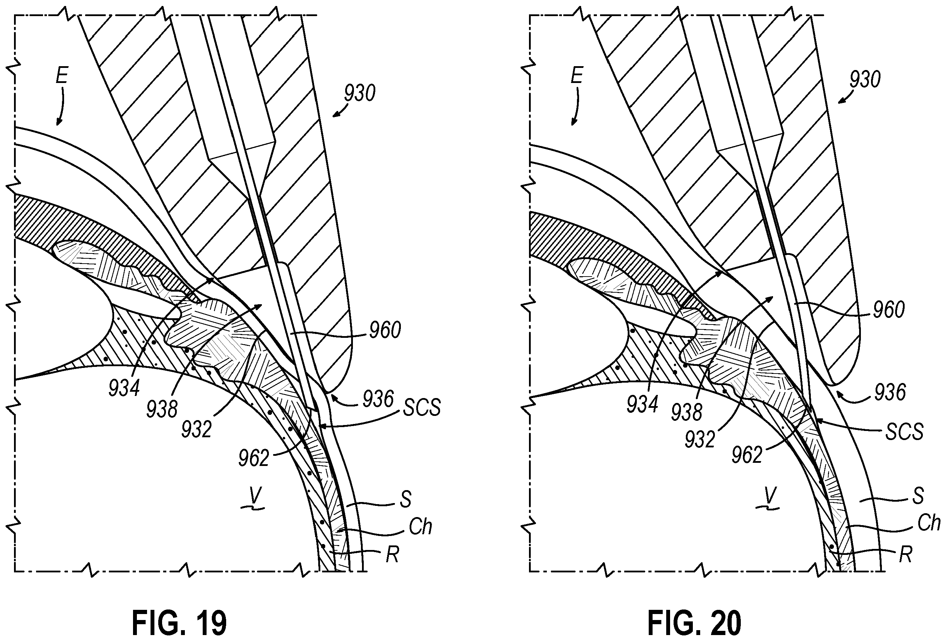

A needle (960) extends distally from head (930). A portion of needle (960) is positioned in a laterally presented recess or trough (938) that is formed in distal face (932). Needle (960) has a sharp, beveled tip (962) that is exposed relative to head (930). Needle (960) may comprise stainless steel and/or any other suitable material(s). In the present embodiment, needle (960) is resiliently flexible, such that the region of needle (960) that is exposed relative to head (930) may deform laterally (e.g., as described below with reference to FIG. 19). In some scenarios, a portion of needle (960) may flex laterally out of trough (938) and away from trough (938). Trough (938) may thus provide clearance for lateral deformation of needle (960). Needle (960) may be approximately 30 gauge or any other suitable size. In this embodiment, needle (960) is straight and extends along a needle axis (NA) that is parallel with the longitudinal axis of shaft (912). In some versions, the needle axis (NA) is coaxial with the longitudinal axis of shaft (912). In some other versions, the needle axis is laterally offset from (yet still parallel with) the longitudinal axis of shaft (912). In the present embodiment, the position of needle (960) relative to shaft (910) is fixed, such that needle (960) does not translate longitudinally relative to shaft (910). In some other versions, needle (960) is translatable relative to shaft (910) (e.g., between a proximal retracted position and a distal exposed position).

A flexible conduit (970) extends from shaft (910) and provides a path for fluid communication with the lumen of needle (960) as will be described in greater detail below. Flexible conduit (970) may comprise a transparent flexible tube or may take any other suitable form. The interior of shaft (910) may define a lumen providing a pathway for fluid communication from flexible conduit (970) to needle (960). In some variations, flexible conduit (970) is received such a lumen and connects with a hub at the proximal end of needle (960). In some other variations, flexible conduit (970) distally terminates in the lumen in shaft (910) and needle (960) proximally terminates in shaft (910), such that fluid is communicated from flexible conduit (970) to needle (960) via the lumen. Alternatively, flexible conduit (970) may be fluidically coupled with needle (960) in any other suitable fashion.

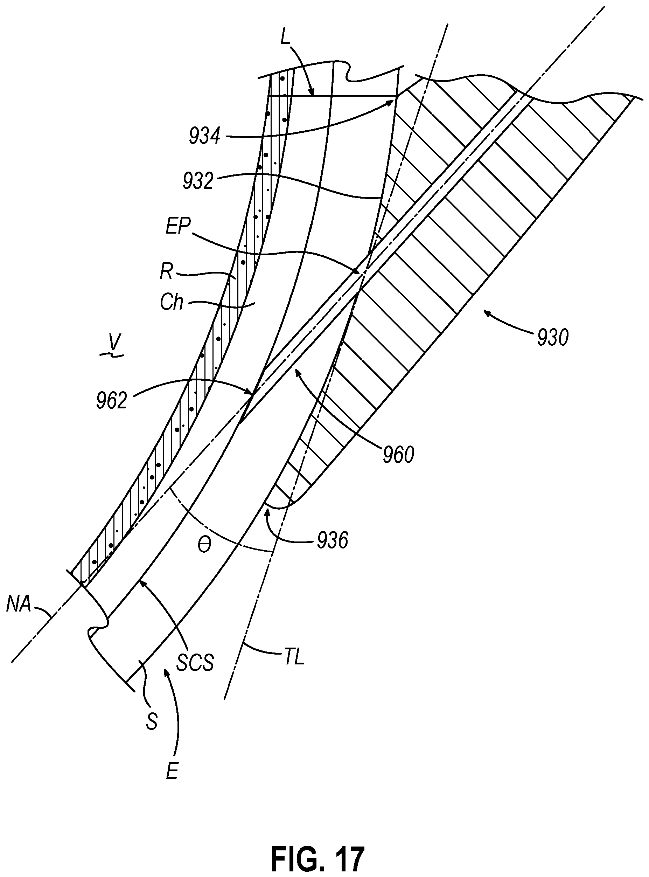

As shown in FIG. 17, instrument (900) may be positioned in relation to an eye (E) such that distal face (932) of head (930) is fully seated against the exterior surface of the eye (E). Needle (960) enters the sclera (S) at an entry point (EP) that is a predetermined distance from the limbus (L) of the eye (E). By way of example only, this predetermined distance from the limbus (L) to the entry point (EP) ranges from approximately 3.0 mm to approximately 4.0 mm; or may be approximately 3.5 mm.

In some scenarios, this predetermined distance from the limbus (L) to the entry point (EP) is achieved by the operator aligning upper edge region (934) of head (930) along the limbus (L), such that upper edge region (934) serves as an indexing feature. In addition, or in the alternative, this predetermined distance may also be achieved using marking prongs (922). The spacing between marking prongs (922) may be selected to indicate the appropriate distance between the limbus (L) and the entry point (EP), such that marking assembly (920) may be used like calipers. In some such scenarios, the operator may first press marking prongs (922) against a pad having biocompatible ink to thereby ink the prongs (922); then press the inked marking prongs (922) against the eye (E) to thereby mark the entry point (EP) with ink. In some other scenarios, the operator may press marking prongs (922) against the eye (E), without ink on marking prongs (922), with sufficient force to cause a marking indentation in the sclera (S) (without piercing the sclera (S)). Alternatively, marking prongs (922) may be used in any other suitable fashion. Alternatively, still, the entry point (EP) may be marked in any other suitable fashion.

Still referring to the arrangement shown in FIG. 17, due to the structural configuration and arrangement of distal face (932) and needle (960), the needle axis (NA) defines a predetermined angle (Θ) with a tangent line (TL) at the entry point (EP). The tangent line (TL) is tangent to the exterior surface of the eye (E) at the entry point (EP). In the present example, this predetermined angle (Θ) is oblique. By way of example only, the angle (Θ) may range from approximately 0 degrees to approximately 40 degrees; or may be approximately 5 degrees. While the angle (Θ) is fixed in the present example, other versions may allow the operator to selectively vary the angle (Θ), as described in various examples provided above.

With distal face (932) of head (930) fully seated against the exterior surface of the eye (E), needle (960) passes fully through the sclera (S) such that tip (962) is positioned in the suprachoroidal space (SCS). The length of needle (960) that is exposed relative to distal face (932), in view of the angle (Θ), is selected to allow tip (962) to reach the suprachoroidal space (SCS) without penetrating the choroid (Ch). However, in some scenarios, tip (962) may incidentally engage the choroid (Ch) without passing fully through the choroid (Ch) and reaching the subretinal space between the choroid (Ch) and the retina (R). By way of example only, the length of needle (960) that is exposed relative to distal face (932) may range from approximately 0.5 mm to approximately 5.0 mm; or may be approximately 1.25 mm. The length of needle (960) that is exposed relative to distal face (932), in combination with the oblique angle (Θ) at which needle (960) enters the eye (E), may reduce the risk of needle (960) undesirably traversing the choroid (Ch). In other words, providing an oblique angle (Θ) for entry of needle (960) may provide less risk of undesirable choroid (Ch) penetration as compared to the risk of undesirable choroid (Ch) penetration that may be presented by a perpendicular angle (Θ) for entry of needle (960). Similarly, the length of needle (960) that is exposed relative to distal face (932), in combination with the oblique angle (Θ) at which needle (960) enters the eye (E), may reduce the risk of needle (960) only reaching an insertion depth where tip (962) remains in the sclera (S), without reaching the suprachoroidal space (SCS). The configuration and orientation of needle (960) may thus accommodate different sclera (S) thicknesses in different patients, with little to no risk of needle (960) being inserted to a depth that is too shallow (i.e., not reaching the suprachoroidal space (SCS)) or too deep (i.e., passing completely through the choroid (Ch) and perhaps even the retina (R)).

In the present example, the bevel angle of tip (962) is selected to promote communication of fluid out from tip (962) into the suprachoroidal space (SCS). By way of example only, this bevel angle may range from approximately 8 degrees to approximately 20 degrees; or may be approximately 14 degrees. Tip (962) may have a tri-bevel configuration or any other suitable configuration. The bevel of tip (962) in the present example is angularly oriented to face the choroid (C), to thereby promote communication of fluid to the suprachoroidal space (SCS) as the fluid exits tip (962) when tip is positioned at or near the suprachoroidal space (SCS).

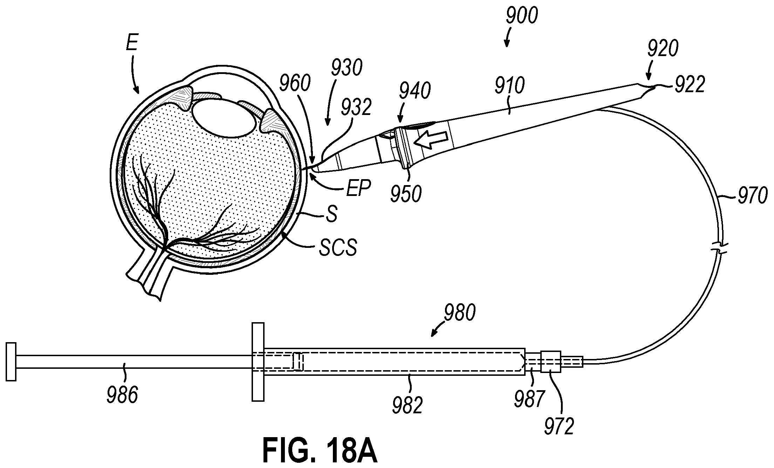

FIGS. 18A-18C show instrument (900) coupled with a syringe (980) for use in a procedure to deliver fluid (e.g., therapeutic agent, etc.) to an eye (E). Syringe (980) of this example includes a conventional syringe, such that syringe (980) includes a barrel (982) containing fluid, a distal luer fitting (984), and a plunger (986) that is slidably disposed in barrel (982). Conduit (970) includes a luer fitting (972) that removably couples with luer fitting (984) of syringe (980). An operator may thus drive fluid from barrel (982) of syringe (980) into conduit (970), and thereby drive the fluid to needle (960) of instrument (900), by advancing plunger (986) distally relative to barrel (982).

As shown in FIG. 18A, the operator may initially position instrument (900) relative to the eye (E) by positioning distal tip (962) of needle (960) at the desired entry point (EP). As noted above, marking assembly (920) may be used like calipers to mark the entry point (EP) relative to the limbus (L) before the operator positions distal tip (962) of needle (960) at the entry point (EP). At the stage shown in FIG. 18A, the operator may refrain from fully advancing needle (960) through the sclera (S). For instance, the operator may just initially penetrate the sclera (S) with distal tip (962) at this stage, or minimally contact the sclera (S) with distal tip (962), to maintain the position of distal tip (962) at the entry point (EP) before fully advancing distal tip (962) into the eye (E) as described below. At this stage, head (930) is still spaced away from the eye (E), such that the only portion of instrument (900) contacting the eye (E) is needle (960).

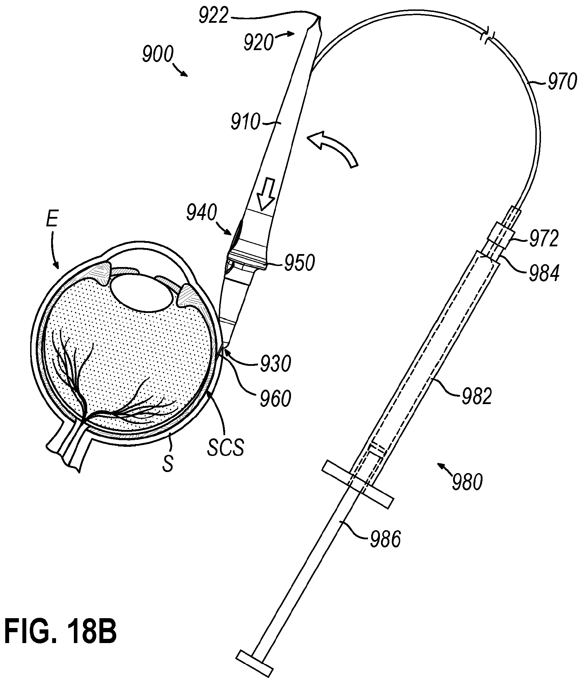

Once the operator has appropriately positioned distal tip (962) at the entry point (E), the operator may then pivot instrument (900) relative to the eye (E), with the interface of needle (960) and the entry point (EP) providing the pivot point for such pivotal motion. The operator may pivot instrument (900), while visually observing instrument (900) along the length of shaft (910) from the proximal end, until instrument (900) reaches a point where upper edge region (934) of distal face (932) is generally aligned with the limbus (L). As noted above, concave face (942) of guidance feature (940) may facilitate visual observation of upper edge region (934) of distal face (932) in relation to the limbus (L) during this pivotal movement. Upon reaching the appropriate pivotal alignment of instrument (900) in relation to the eye (E), the operator may complete distal advancement of instrument (900) relative to the eye (E) until distal face (932) is fully seated against the eye (E), as shown in FIG. 18B. With distal face (932) fully seated against the eye (E), needle (960) may be positioned such that distal tip (962) is located precisely at (or substantially at) the suprachoroidal space (SCS) as shown in FIG. 17. In some scenarios, as described below with reference to FIG. 19, the sclera (S) may deform during the transition from the state shown in FIG. 18A to the state shown in FIG. 18B. In some other scenarios, as described below with reference to FIG. 20, the distal portion of needle (960) may deform during the transition from the state shown in FIG. 18A to the state shown in FIG. 18B. In still other scenarios, both the sclera (S) and the distal portion of needle (960) may deform during the transition from the state shown in FIG. 18A to the state shown in FIG. 18B.

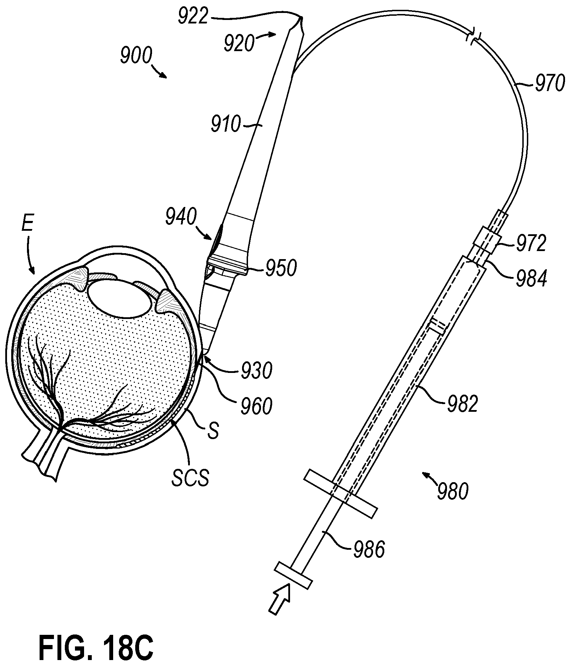

With distal tip (962) located precisely at (or substantially at) the suprachoroidal space (SCS) as shown in FIG. 17, the operator may then advance plunger (986) distally relative to barrel (982), as shown in FIG. 18C. Such advancement of plunger (986) may drive fluid from barrel (982) through flexible conduit (970), and further through needle (960), such that the fluid ultimately exits distal tip (962) into the suprachoroidal space (SCS).

In the present example, since the needle axis (NA) is oriented at an angle (Θ) that is not perpendicular to the suprachoroidal space (SCS), the fluid delivered via distal tip (962) may diffuse more easily within the suprachoroidal space (SCS) than the fluid might otherwise diffuse in a scenario where the fluid is delivered via a needle that is oriented perpendicular to the suprachoroidal space (SCS). In other words, the oblique angle (Θ) of the needle axis (NA) may promote diffusion of the delivered fluid within the suprachoroidal space (SCS). The oblique angle (Θ) of the needle axis (NA) may also reduce a risk of the choroid (C) being acutely stretched at the injection site, which might otherwise occur in scenarios where the fluid is delivered via a needle that is oriented perpendicular to the suprachoroidal space (SCS). Moreover, the oblique angle (Θ) of the needle axis (NA) may reduce the risk of the retina (R) being undesirably penetrated by distal tip (962).

While instrument (900) is described above as being coupled with syringe (980) via conduit (970), instrument (900) may instead be coupled with any other suitable kind of fluid delivery device or system. For instance, instrument (900) may instead be coupled with an automated fluid delivery system. Instrument (900) may also incorporate any of the various other features described herein, including but not limited to the integral plunger (20) of instrument (10), actuator (250) of head (200) and associated components, adjustment member (470) of head (400), electrodes (504) of head (500) and associated components, and/or distal projections (830, 840) of head (800).

In the present example, distal tip (962) never penetrates the retina (R) and never reaches the vitreous chamber (V). Thus, distal tip (962) penetrates no further than the suprachoroidal space (SCS) or the choroid (Ch). In some other variations, at least a portion of distal tip (962) penetrates the retina (R). Moreover, distal tip (962) may reach the vitreous chamber (V) in some variations.

The configuration of instrument (900) as described above with reference to FIGS. 16-17, and the operation of instrument as described above with reference to FIGS. 18A-18C, may facilitate use of instrument (900) in eyes (E) having different sclera (S) thicknesses. In other words, instrument (900) may be just as suitable for use in an eye (E) having a thick sclera (S) as it is for use in an eye (E) having a thin sclera (S). This is illustrated in FIGS. 19-20. In particular, FIG. 19 shows instrument (900) being used in an eye (E) having a relatively thin sclera (S). In such an eye (E), when instrument (900) is pivoted from the position shown in FIG. 18A to the position shown in FIG. 18B, the resilience of needle (960) may overcome the resilience of the sclera (S), such that the distal end of needle (960) may cause some degree of deformation of the adjacent region of the sclera (S), without causing deformation of needle (960). Tip (962) of needle (960) may still reach, and remain in, the suprachoroidal space (SCS) to administer a therapeutic agent, etc., to the suprachoroidal space (SCS). As shown in FIG. 19, tip (962) does not traverse the choroid (Ch) during the procedure. In some cases, tip (962) does not even engage the choroid (Ch) during the procedure.

FIG. 20 shows instrument (900) being used in an eye (E) having a relatively thick sclera (S). In such an eye (E), when instrument (900) is pivoted from the position shown in FIG. 18A to the position shown in FIG. 18B, the resilience of the sclera (S) may overcome the resilience of needle (960), such that the sclera (S) may cause the distal end of needle (960) to flexibly deform, without causing deformation of sclera (S). Tip (962) of needle (960) may still reach, and remain in, the suprachoroidal space (SCS) to administer a therapeutic agent, etc., to the suprachoroidal space (SCS). As shown in FIG. 20, tip (962) does not traverse the choroid (Ch) during the procedure. In some cases, tip (962) does not even engage the choroid (Ch) during the procedure.

While FIG. 19 shows deformation of the sclera (S) without deformation of needle (960), and FIG. 20 shows deformation of needle (960) without deformation of the sclera (S), there may be scenarios (e.g., where the sclera (S) has a thickness somewhere between the thickness shown in FIG. 19 and the thickness shown in FIG. 20) where the sclera (S) and needle (960) both deform to some degree. Again, regardless of whether the sclera (S) deforms, needle (960) deforms, or both the sclera (S) and needle (960) deform, tip (962) of needle (960) may still reach, and remain in, the suprachoroidal space (SCS) to administer a therapeutic agent, etc., to the suprachoroidal space (SCS). Moreover, regardless of whether the sclera (S) deforms, needle (960) deforms, or both the sclera (S) and needle (960) deform, tip (962) of needle (960) may avoid imparting undue trauma to the choroid (Ch). Thus, needle (960) may reach, and remain in, the suprachoroidal space (SCS) to administer a therapeutic agent, and avoid imparting undue trauma to the choroid (Ch), regardless of the thickness of the sclera (S). The length of needle (960) that is exposed relative to distal face (932), in combination with the oblique angle (Θ) at which needle (960) enters the eye (E), in further combination with the resilient flexibility of needle (960) may further reduce the risk of tip (962) failing to reach the suprachoroidal space (SCS) (e.g., where tip (962) may otherwise fail to fully traverse the sclera (S)).

### III. Miscellaneous

To the extent that several examples herein are described in the context of a cannula guide being positioned near an already-formed scleral incision (23), it should be understood that other kinds of procedures may be employed. For instance, in some variations of the procedures described herein, the cannula guide may be secured to the eye (20) first; and then the scleral incision (23) may be formed after the cannula guide has been secured to the eye (20). Other suitable steps and sequences that may be carried out in procedures that include a combination of a scleral incision (23) and a cannula guide will be apparent to those skilled in the art in view of the teachings herein.

It should be understood that any of the versions of the instruments described herein may include various other features in addition to or in lieu of those described above.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention as defined by the claims. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus (900) for delivering fluid from a syringe to a suprachoroidal space (SCS), comprising:
(a) a shaft (910) having:
(i) a proximal end (920),
(ii) a distal end, and
(iii) a fluid pathway; and
(b) a head (930) at the distal end of the shaft (910), the head (930) including:
(i) a distal face (932), the distal face (932) being configured to engage an exterior surface of a patient's eye (E) at an anterior region of the patient's eye (E), the distal face (932) having a concave curvature contoured to complement a curvature of the patient's eye (E), and
(ii) a needle (960), the needle (960) being configured to extend distally from the distal face (932), the needle (960) having a length that is exposed relative to the distal face (932) that is sufficient to enter a patient's eye (E) at an entry point (EP) and extend through a sclera layer (S) of the patient's eye (E) and thereby position at least a portion of a distal tip of the needle (960) in the suprachoroidal space (SCS) of the patient's eye (E) at an anterior region of the patient's eye without passing fully through the choroid (Ch) while the distal face (932) is seated against the patient's eye, the needle (960) being in fluid communication with the fluid pathway such that the needle (960) is operable to deliver fluid from the fluid pathway into the suprachoroidal space (SCS) of the patient's eye, **characterized in that**:
the needle (960) is resiliently flexible such that the region of the needle (960) that is exposed relative to the distal face (932) of the head (930) can deform laterally such that the sclera (S) may cause the distal end of needle (960) to flexibly deform, without causing deformation of sclera (S).

2. The apparatus of claim 1, the distal face (932) having an edge (934), the edge (934) defining an indexing feature, the indexing feature having a curvature configured to complement a curvature of the limbus of the patient's eye to thereby position the needle (960) at a predetermined location in relation to the limbus of the patient's eye.

3. The apparatus of claim 1 or 2, the needle (960) being longitudinally fixed relative to the distal face (932).

4. The apparatus of any of claims 1 through 3, the distal face (932) having a three-dimensional concave curvature.

5. The apparatus of any of claims 1 through 4, the shaft (910) defining a longitudinal axis, the needle (960) extending along an exit axis that is parallel with the longitudinal axis of the shaft (910).

6. The apparatus of any of claims 1 through 5, the needle (960) having a straight configuration.

7. The apparatus of any of claims 1 through 6, the needle (960) having a beveled distal tip (962), the beveled distal tip (962) defining a bevel angle, the bevel angle being configured to be parallel with boundaries of layers of the patient's eye as the needle (960) is inserted into the patient's eye.

8. The apparatus of any of claims 1 through 7, the fluid pathway comprising a flexible conduit (970), and further comprising a syringe (980) coupled with the flexible conduit (970), the syringe (980) being spaced apart from the shaft (910) via the flexible conduit (970).

9. The apparatus of any of claims 1 through 8, the shaft (910) further including a laterally facing guidance feature, the laterally facing guidance feature (940) being configured to complement a landmark of the patient's eye to thereby position the needle at a predetermined orientation in relation to the patient's eye, the laterally facing guidance feature (940) including a concave face (942) configured to complement the curvature of the landmark of the patient's eye.

10. The apparatus of any of claims 1 through 9, the shaft (910) further including a marking assembly (922), the marking assembly (922) being configured to mark a needle entry point in relation to a landmark of the patient's eye.

11. The apparatus of claim 10, the marking assembly (922) being positioned at the proximal end (920) of the shaft.

12. The apparatus of any of claims 10 through 11, the marking assembly including two prongs (922) fixedly spaced apart from each other by a predetermined distance.

13. The apparatus of any of claims 10 through 12, the marking assembly (922) being configured to mark a needle entry point in relation to a limbus of the patient's eye.

14. The apparatus of any preceding claim, wherein the needle (960) extends along a needle axis (NA) that defines a predetermined angle (θ) with a tangent line (TL) of the distal face (932) at the entry point (EP), wherein the predetermined angle (θ) is in the range from 0 to 40 degrees, and the length of the needle (960) that is exposed relative to the distal face (932) is in the range from 0.5mm to 5.0mm

## Patentansprüche

1. Gerät (900) zum Abgeben von Fluid aus einer Spritze in einen suprachoroidalen Raum (SCS), umfassend:
(a) einen Schaft (910) mit:
(i) einem proximalen Ende (920),
(ii) einem distalen Ende und
(iii) einem Fluidweg; und
(b) einen Kopf (930) am distalen Ende des Schafts (910), wobei der Kopf (930) Folgendes einschließt:
(i) eine distale Fläche (932), wobei die distale Fläche (932) dazu konfiguriert ist, an einer äußeren Oberfläche eines Patientenauges (E) in einem anterioren Bereich des Patientenauges (E) anzuliegen, wobei die distale Fläche (932) eine konkave Krümmung aufweist, die konturiert ist, um zu einer Krümmung des Patientenauges (E) komplementär zu sein, und
(ii) eine Nadel (960), wobei die Nadel (960) dazu konfiguriert ist, sich distal von der distalen Fläche (932) zu erstrecken, wobei die Nadel (960) eine Länge aufweist, die relativ zur distalen Fläche (932) freiliegt und die ausreicht, um an einem Eintrittspunkt (EP) in ein Patientenauge (E) einzutreten und sich durch eine Skleraschicht (S) des Patientenauges (E) zu erstrecken und dadurch mindestens einen Abschnitt einer distalen Spitze der Nadel (960) im suprachoroidalen Raum (SCS) des Patientenauges (E) in einem anterioren Bereich des Patientenauges zu positionieren, ohne die Choroidea (Ch) vollständig zu durchdringen, während die distale Fläche (932) am Patientenauge anliegt, wobei die Nadel (960) in Fluidverbindung mit dem Fluidweg steht, sodass die Nadel (960) betätigt werden kann, um Fluid aus dem Fluidweg in den suprachoroidalen Raum (SCS) des Patientenauges abzugeben, **dadurch gekennzeichnet, dass**:
die Nadel (960) elastisch flexibel ist, sodass sich der Bereich der Nadel (960), der relativ zur distalen Fläche (932) des Kopfs (930) freiliegt, lateral verformen kann, so dass die Sklera (S) eine flexible Verformung des distalen Endes der Nadel (960) bewirken kann, ohne dass es zu einer Verformung der Sklera (S) kommt.

2. Gerät nach Anspruch 1, wobei die distale Fläche (932) eine Kante (934) aufweist, wobei die Kante (934) ein Indexierungsmerkmal definiert, wobei das Indexierungsmerkmal eine Krümmung aufweist, die dazu konfiguriert ist, zu einer Krümmung des Limbus des Patientenauges komplementär zu sein, um dadurch die Nadel (960) an einer vorbestimmten Stelle in Bezug auf den Limbus des Patientenauges zu positionieren.

3. Gerät nach Anspruch 1 oder 2, wobei die Nadel (960) relativ zur distalen Fläche (932) in Längsrichtung fixiert ist.

4. Gerät nach einem der Ansprüche 1 bis 3, wobei die distale Fläche (932) eine dreidimensionale konkave Krümmung aufweist.

5. Gerät nach einem der Ansprüche 1 bis 4, wobei der Schaft (910) eine Längsachse definiert, wobei sich die Nadel (960) entlang einer Austrittsachse erstreckt, die parallel zur Längsachse des Schafts (910) verläuft.

6. Gerät nach einem der Ansprüche 1 bis 5, wobei die Nadel (960) eine gerade Konfiguration aufweist.

7. Gerät nach einem der Ansprüche 1 bis 6, wobei die Nadel (960) eine abgeschrägte distale Spitze (962) aufweist, wobei die abgeschrägte distale Spitze (962) einen Schrägwinkel definiert, wobei der Schrägwinkel dazu konfiguriert ist, parallel zu Grenzen von Schichten des Patientenauges zu verlaufen, wenn die Nadel (960) in das Patientenauge eingeführt wird.

8. Gerät nach einem der Ansprüche 1 bis 7, wobei der Fluidweg eine flexible Leitung (970) umfasst und ferner eine Spritze (980) umfasst, die mit der flexiblen Leitung (970) gekoppelt ist, wobei die Spritze (980) über die flexible Leitung (970) vom Schaft (910) beabstandet ist.

9. Gerät nach einem der Ansprüche 1 bis 8, wobei der Schaft (910) ferner ein lateral ausgerichtetes Führungsmerkmal einschließt, wobei das lateral ausgerichtete Führungsmerkmal (940) dazu konfiguriert ist, zu einem Orientierungspunkt des Patientenauges komplementär zu sein, um dadurch die Nadel in einer vorbestimmten Ausrichtung in Bezug auf das Patientenauge zu positionieren, wobei das lateral ausgerichtete Führungsmerkmal (940) eine konkave Fläche (942) einschließt, die dazu konfiguriert ist, zu der Krümmung des Orientierungspunkts des Patientenauges komplementär zu sein.

10. Gerät nach einem der Ansprüche 1 bis 9, wobei der Schaft (910) ferner eine Markierungsanordnung (922) einschließt, wobei die Markierungsanordnung (922) dazu konfiguriert ist, einen Nadeleintrittspunkt in Bezug auf einen Orientierungspunkt des Patientenauges zu markieren.

11. Gerät nach Anspruch 10, wobei die Markierungsanordnung (922) am proximalen Ende (920) des Schafts positioniert ist.

12. Gerät nach einem der Ansprüche 10 bis 11, wobei die Markierungsanordnung zwei Zinken (922) einschließt, die um einen vorbestimmten Abstand fest voneinander beabstandet sind.

13. Gerät nach einem der Ansprüche 10 bis 12, wobei die Markierungsanordnung (922) dazu konfiguriert ist, einen Nadeleintrittspunkt in Bezug auf einen Limbus des Patientenauges zu markieren.

14. Gerät nach einem der vorhergehenden Ansprüche, wobei sich die Nadel (960) entlang einer Nadelachse (NA) erstreckt, die einen vorbestimmten Winkel (θ) mit einer Tangentenlinie (TL) der distalen Fläche (932) am Eintrittspunkt (EP) definiert, wobei der vorbestimmte Winkel (θ) im Bereich von 0 bis 40 Grad liegt und die Länge der Nadel (960), die relativ zur distalen Fläche (932) freiliegt, im Bereich von 0,5 mm bis 5,0 mm liegt.

## Revendications

1. Appareil (900) d'administration d'un fluide à partir d'une seringue dans un espace suprachoroïdien (SCS), comprenant :
(a) une tige (910) ayant :
(i) une extrémité proximale (920),
(ii) une extrémité distale, et
(iii) une voie de passage de fluide ; et
(b) une tête (930) au niveau de l'extrémité distale de la tige (910), la tête (930) comprenant :
(i) une face distale (932), la face distale (932) étant conçue pour venir en prise avec une surface extérieure d'un œil d'un patient (E) au niveau d'une région antérieure de l'œil du patient (E), la face distale (932) ayant une courbure concave profilée pour épouser une courbure de l'œil du patient (E), et
(ii) une aiguille (960), l'aiguille (960) étant conçue pour s'étendre distalement à partir de la face distale (932), l'aiguille (960) ayant une longueur qui est exposée par rapport à la face distale (932) qui est suffisante pour entrer dans un œil d'un patient (E) au niveau d'un point d'entrée (EP) et s'étendre à travers une couche de sclère (S) de l'œil du patient (E) et positionner ainsi au moins une partie d'une pointe distale de l'aiguille (960) dans l'espace suprachoroïdien (SCS) de l'œil du patient (E) au niveau d'une région antérieure de l'œil du patient sans traverser complètement la choroïde (Ch) tandis que la face distale (932) repose contre l'œil du patient, l'aiguille (960) étant en communication fluidique avec la voie de passage de fluide de telle sorte que l'aiguille (960) est apte à administrer un fluide à partir de la voie de passage de fluide dans l'espace suprachoroïdien (SCS) de l'œil du patient, **caractérisé en ce que** :
l'aiguille (960) est élastiquement flexible de telle sorte que la région de l'aiguille (960) qui est exposée par rapport à la face distale (932) de la tête (930) peut se déformer latéralement de telle sorte que la sclère (S) peut provoquer la déformation de manière flexible de l'extrémité distale de l'aiguille (960), sans provoquer la déformation de la sclère (S).

2. Appareil selon la revendication 1, la face distale (932) ayant un bord (934), le bord (934) définissant un élément d'indexation, l'élément d'indexation ayant une courbure conçue pour épouser une courbure du limbe de l'œil du patient pour ainsi positionner l'aiguille (960) au niveau d'un emplacement prédéterminé par rapport au limbe de l'œil du patient.

3. Appareil selon la revendication 1 ou 2, l'aiguille (960) étant fixée longitudinalement par rapport à la face distale (932).

4. Appareil selon l'une quelconque des revendications 1 à 3, la face distale (932) ayant une courbure concave tridimensionnelle.

5. Appareil selon l'une quelconque des revendications 1 à 4, la tige (910) définissant un axe longitudinal, l'aiguille (960) s'étendant le long d'un axe de sortie qui est parallèle à l'axe longitudinal de la tige (910).

6. Appareil selon l'une quelconque des revendications 1 à 5, l'aiguille (960) ayant une configuration rectiligne.

7. Appareil selon l'une quelconque des revendications 1 à 6, l'aiguille (960) ayant une pointe distale biseautée (962), la pointe distale biseautée (962) définissant un angle de biseau, l'angle de biseau étant conçu pour être parallèle aux limites des couches de l'œil du patient lorsque l'aiguille (960) est insérée dans l'œil du patient.

8. Appareil selon l'une quelconque des revendications 1 à 7, la voie de passage de fluide comprenant un conduit flexible (970), et comprenant en outre une seringue (980) accouplée au conduit flexible (970), la seringue (980) étant espacée de la tige (910) par l'intermédiaire du conduit flexible (970).

9. Appareil selon l'une quelconque des revendications 1 à 8, la tige (910) comprenant en outre un élément de guidage orienté latéralement, l'élément de guidage orienté latéralement (940) étant conçu pour épouser un point de repère de l'œil du patient pour ainsi positionner l'aiguille selon une orientation prédéterminée par rapport à l'œil du patient, l'élément de guidage orienté latéralement (940) comprenant une face concave (942) conçue pour épouser la courbure du point de repère de l'œil du patient.

10. Appareil selon l'une quelconque des revendications 1 à 9, la tige (910) comprenant en outre un ensemble de marquage (922), l'ensemble de marquage (922) étant conçu pour marquer un point d'entrée d'aiguille par rapport à un point de repère de l'œil du patient.

11. Appareil selon la revendication 10, l'ensemble de marquage (922) étant positionné au niveau de l'extrémité proximale (920) de la tige.

12. Appareil selon l'une quelconque des revendications 10 à 11, l'ensemble de marquage comprenant deux dents (922) espacées de manière fixe l'une de l'autre d'une distance prédéterminée.

13. Appareil selon l'une quelconque des revendications 10 à 12, l'ensemble de marquage (922) étant conçu pour marquer un point d'entrée d'aiguille par rapport à un limbe de l'œil du patient.

14. Appareil selon une quelconque revendication précédente, dans lequel l'aiguille (960) s'étend le long d'un axe d'aiguille (NA) qui définit un angle (θ) prédéterminé avec une ligne tangente (TL) de la face distale (932) au niveau du point d'entrée (EP), dans lequel l'angle (θ) prédéterminé est dans la plage allant de 0 à 40 degrés, et la longueur de l'aiguille (960) qui est exposée par rapport à la face distale (932) est dans la plage allant de 0,5 mm à 5,0 mm.
